# EUROPEAN PATENT APPLICATION

(11) **EP 2 468 872 A1**
(43) Date of publication of application: **27.06.2012**
(21) Application number: 10016035.7
(22) Date of filing: 23.12.2010
(51) Int. Cl.: C12N 15/82

(54) **Method for producing mullerian inhibitor substance in plants**

(71) Applicant: Philip Morris Products S.A., 2000 Neuchâtel (CH)
(72) Inventor: The designation of the inventor has not yet been filed
(74) Representative: Lloyd, John Scott

(57) **Abstract**

The present invention provides, in one aspect, a method for producing Mullerian Inhibitor Substance in a plant comprising incubating or growing a plant into which has been introduced or infiltrated a nucleic acid construct comprising, consisting or consisting essentially of a nucleic acid sequence encoding a Mullerian Inhibitor Substance fusion protein that comprises a fusion protein partner that induces the formation of a protein body in a plant, preferably, wherein the step of introducing or infiltrating the plant is performed prior to the incubating or growing step.

## Description

### FIELD OF THE INVENTION

The present invention relates to a method for producing Mullerian Inhibitor Substance (MIS) in a plant by accumulation thereof in a protein body. Nucleic acid sequences, nucleic acid constructs, vectors, expression vectors and the like for carrying out the method are also disclosed.

### BACKGROUND OF THE INVENTION

Proteins of the TGF-beta family mediate important embryogenic and immune functions including chemotaxis, production of extracellular matrix, regulation of cell growth and differentiation, and development and regulation of the immune system. Thus, these molecules have applications in a number of different treatments if available in sufficient quantities. Mullerian Inhibiting Substance (MIS), which is also known as Anti-Mullerian Hormone (AMH) is a member of the TGF-beta family. MIS is a 140 kDa dimeric glycoprotein hormone. In common with other TGF-beta proteins, MIS is synthesized as a large precursor with a short signal sequence followed by the pre-pro hormone that forms homodimers. Prior to secretion, the mature hormone undergoes glycosylation and dimerisation to produce a 140-kDa dimer of identical disulphide-linked 70-kDa monomer subunits; each monomer contains an N-terminal domain (also called the "pro" region) and a C-terminal domain (also called the "mature" region). Between 5-20% of MIS is then cleaved at a specific site by a KEX serine protease between the N-terminal domain (the pro region) and the C-terminal domain (the mature region) of the 70-kDa monomer during cytoplasmic transit, to form two polypeptides of 58 kDa (pro region) and 12 kDa (mature region). These two parts of the monomer remain in non-covalent attachment. The human gene coding for MIS has been sequenced and isolated, and is located on the short arm of chromosome 19 (Proc. Natl. Acad. Sci. (1986), 83, 5464-5468). The structure of a specific receptor for MIS has also been isolated and characterized.

MIS has an important role in sexual differentiation during development. MIS is produced by the Sertoli cells of the testis in the male, and by ovarian granulosa cells in the female. During fetal development in males, secretion of MIS from testicular Sertoli cells is essential for the regression of the Mullerian ducts, and thus the normal development of the male reproductive tract. The Mullerian ducts are the primordium for the uterus, Fallopian tubes, and upper part of the vagina in the female. In the male, secretion of MIS by the Sertoli cells commences during embryogenesis and continues throughout life. In the female, serum MIS is maintained at relatively low levels when compared to the male. In mice, ablation of AMH function causes increased loss of ovarian follicles and premature cessation of ovarian cycling. MIS may be an effective therapy for cancer, respiratory distress syndrome, and fertility/contraception. Any cancer comprising MIS receptors may be treatable with MIS. These cancers include vulvar carcinoma, some ocular melanomas, ovarian epithelial cancer, prostate cancer, and breast cancer.

The biochemical, technical, and economic limitations of existing prokaryotic and eukaryotic expression systems have created substantial interest in developing new and optimised production systems for heterologous proteins. To that end, plant expression systems can be used to produce recombinant proteins. However, a number of variables, including crop species selection, tissues choice, expression and recovery strategies and posttranslational processing have to be taken into consideration during the development and commercialization of a plant based production system. Accordingly, the development of a plant based production system is not straightforward and there is no certainty that the system that is eventually developed will be one that results in the effective production of the selected protein, especially on a commercial scale. In particular, problems are often encountered when purifying the recombinant protein from the plant expression system. This represents one of the most significant bottlenecks in recombinant protein production in plants. Protein purification from plants is a difficult task due to the complexity of the plant system. Plant solids are typically large, dense and relatively elevated at about 10-20% by weight. All of these problems are particularly acute in the context of the industrial production of recombinant proteins in plants, where multiple or complex steps may render the method unsuitable.

Current production systems for MIS are not capable of producing MIS at the levels required for clinical trials or commercial applications. Recombinant MIS is also a challenging protein to express and produce, especially on a commercially useful scale. The method provided by the present invention meets this need.

### SUMMARY OF THE INVENTION

The present invention provides a method for producing MIS in a plant based expression system, utilising a fusion protein partner that induces the formation of a protein body in a plant. Advantageously, when the protein that induces the formation of a protein body in a plant further comprises one or more non-naturally occurring repeat sequence motifs therein, this can result in the highly efficient expression of MIS in a plant cell which can be about 30 times higher than the expression level of MIS without the additional repeat sequence motifs. Moreover, the efficient expression of MIS in plant protein bodies facilitates the recovery of the recombinant MIS fusion protein and the methods described herein can be used to obtain MIS in a substantially purified form on a commercial scale. The methods are therefore useful for producing substantially purified MIS that is easily scalable for mass production of the protein.

In a first aspect, there is provided a method for producing Mullerian Inhibitor Substance in a plant comprising incubating or growing a plant into which has been introduced or infiltrated a nucleic acid construct comprising, consisting or consisting essentially of a nucleic acid sequence encoding a Mullerian Inhibitor Substance fusion protein that comprises a fusion protein partner that induces the formation of a protein body in a plant.

In a further aspect, there is provided a method for producing MIS in a plant comprising the steps of: (a) introducing into a plant a nucleic acid construct comprising, consisting or consisting essentially of a nucleic acid sequence encoding a protein that induces the formation of a protein body in a plant; and a nucleic acid sequence encoding MIS, wherein said nucleic acid sequences are operably linked to each other; and (b) incubating said plant under conditions that allow for the expression of MIS as a fusion protein in said plant.

In a further aspect, there is provided a method for expressing MIS in a plant comprising the use of a nucleic acid construct comprising, consisting or consisting essentially of a nucleic acid sequence encoding a protein that induces the formation of a protein body in a plant; and a nucleic acid sequence encoding MIS, wherein said nucleic acid sequences are operably linked to each other.

In a further aspect, there is provided a method for expressing MIS in a plant comprising the step of: (a) providing a plant comprising a nucleic acid construct comprising, consisting or consisting essentially of a nucleic acid sequence encoding a protein that induces the formation of a protein body in a plant; and a nucleic acid sequence encoding MIS, wherein said nucleic acid sequences are operably linked to each other; and (b) incubating said plant under conditions that allow for the expression of MIS as a fusion protein in said plant.

In one embodiment, the step of introducing or infiltrating the plant is performed prior to the incubating or growing step.

In one embodiment, the nucleic acid construct used in the method comprises: a first nucleic acid sequence encoding a protein that induces the formation of a protein body in a plant, optionally, further comprising a nucleic acid sequence encoding a non-naturally occurring repeat sequence motif; a second nucleic acid sequence encoding MIS; and optionally a third nucleic acid sequence encoding an amino acid linker in which a peptide bond therein can be specifically cleaved; wherein said first, second and third nucleic acid sequences are operably linked to each other.

In one embodiment or combination of above-mentioned embodiments, said protein that induces the formation of a protein body in a plant is gamma-zein.

In one embodiment or combination of above-mentioned embodiments, the nucleic acid sequence used in the method and encoding the protein that induces the formation of a protein body in a plant further comprises a (fourth) nucleic acid sequence encoding a peptide that directs the fusion protein towards the endoplasmic reticulum of a plant cell, preferably a signal peptide.

In one embodiment or combination of above-mentioned embodiments, the method comprises the additional step of: recovering the protein body comprising the fusion protein from the plant, preferably wherein said step comprises the steps of: (i) homogenising the plant material; (ii) centrifuging the homogenised plant material at low speed, preferably, about 200 x g; (iii) centrifuging the homogenised plant material at a higher speed than step (ii), preferably, about 6000 x g; and (iv) recovering the protein bodies comprising the fusion protein in the pelleted fraction.

In one embodiment or combination of above-mentioned embodiments, the method comprises the further step of solubilising the pelleted fraction comprising the fusion protein, preferably, wherein said solubilisation step comprises the use of a mixture comprising, consisting or consisting essentially of urea, dithiothreitol and (tris(2-carboxyethyl)phosphine).

In one embodiment or combination of above-mentioned embodiments, the method comprises the further step of releasing MIS from said fusion protein, preferably, wherein a protease, preferably, TEV protease, or a protein splicing means, preferably an intein, is used to release MIS from said fusion protein

In one embodiment or combination of above-mentioned embodiments, said protease or said protein splicing means cleaves MIS from the fusion protein to leave less than three, two or one residual amino acids at the cleaved end of MIS. In one embodiment or combination of above-mentioned embodiments, said protease or said protein splicing means cleaves MIS from the fusion protein without leaving any residual amino acids at the cleaved end of MIS.

In one embodiment or combination of above-mentioned embodiments, the method comprises the further step of: purifying the released MIS protein.

In a further aspect, there is provided a nucleic acid construct comprising, consisting or consisting essentially of: a first nucleic acid sequence encoding a protein that induces the formation of a protein body in a plant, optionally, wherein said sequence comprises a nucleic acid sequence encoding a non-naturally occurring repeat sequence motif; a second nucleic acid sequence encoding MIS; and optionally a third nucleic acid sequence encoding an amino acid linker in which a peptide bond therein can be specifically cleaved; wherein said first, second and third nucleic acid sequences are operably linked to each other.

In a further aspect, there is provided a nucleic acid construct comprising: (i) the nucleic acid sequence; and (ii) a regulatory nucleotide sequence that regulates the transcription of said nucleic acid sequence.

In a further aspect, there is provided a vector comprising the nucleic acid sequence or the nucleic acid construct.

In a further aspect, there is provided a fusion protein comprising, consisting or consisting essentially of: (i) an amino acid sequence encoding a protein that induces the formation of a protein body in a plant, optionally, wherein said amino acid sequence further comprises an amino acid sequence encoding a non-naturally occurring repeat sequence motif (ii) optionally an amino acid sequence encoding a linker in which a peptide bond therein can be specifically cleaved; (iii) an amino acid sequence encoding MIS.

In a further aspect, there is provided a plant or plant material derived therefrom comprising the nucleic acid sequence, or the nucleic acid construct, or the vector, or the fusion protein described herein. Suitably, the plant or plant material is a transformed or infiltrated plant or plant material.

In a further aspect, there is provided a plant protein body comprising the fusion protein.

In a further aspect, there is provided the use of the nucleic acid sequence, or the nucleic acid construct, or the vector for expressing and/or producing MIS in a plant cell.

The embodiments and combinations of embodiments described above in relation to the method(s) for producing MIS in a plant are also disclosed as embodiments of the further aspects described above.

Using the methods described herein, MIS can be expressed in a plant at a higher level when fused to a nucleic acid sequence that encodes a protein that induces the formation of a protein body in a plant and optionally also includes one or more non-naturally occurring repeat sequence motifs in the protein that induces the formation of the protein body. The expression level of MIS achieved in plants is significantly higher in the presence of the protein that induces the formation of a protein body and can be even higher when the one or more non-naturally occurring repeat sequence motifs are included therein as compared to the absence of the one or more non-naturally occurring repeat sequence motifs. The high level expression of recombinant MIS in protein bodies can protect the protein from proteolytic and enzymatic activities that may be present in the plant.

The protein bodies comprising recombinant MIS fusion protein can retain their high density which can simplify the recovery of MIS protein.

The downstream recovery and purification protocol of the method can be simpler and less costly than current approaches for preparing recombinant MIS. By way of example, an amino acid linker in which a peptide bond therein can be specifically cleaved is included to allow MIS to be separated from the fusion protein. In one embodiment, the amino acid linker is a cleavage recognition site.

The recombinant MIS protein may be identical in amino acid sequence to the native protein thereby rendering it suitable for use in clinical applications.

The methods can be used for producing substantially purified MIS on an industrial scale.

### BRIEF DESCRIPTION OF THE DRAWINGS

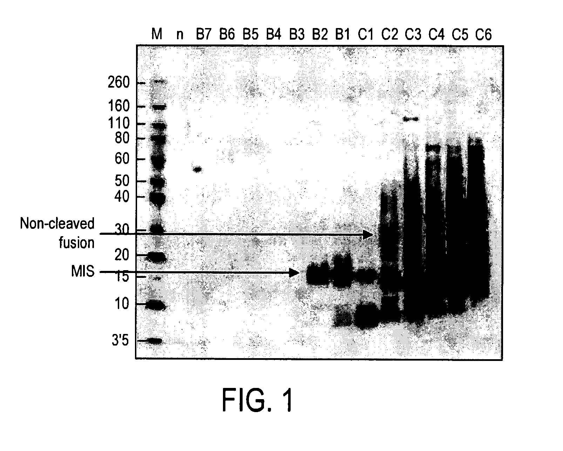

**Figure 1** is an SDS-PAGE gel of purified recombinant MIS obtained according to the present invention. Purified MIS is shown in Lane B2.

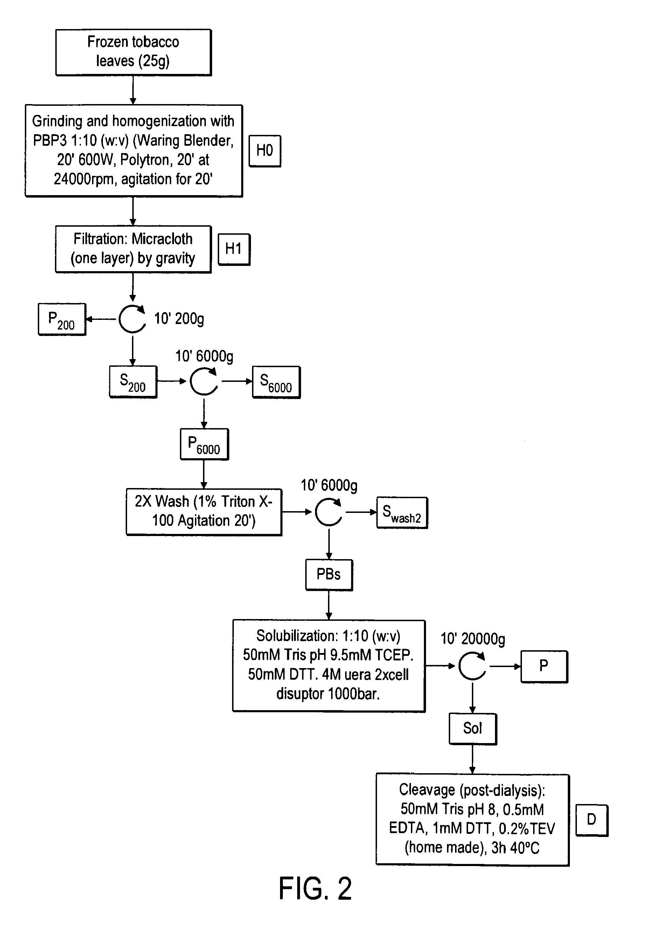

**Figure 2** illustrates one embodiment of the method of the present invention.

### DEFINITIONS

The technical terms and expressions used within the scope of this application are generally to be given the meaning commonly applied to them in the pertinent art of plant and molecular biology. All of the following term definitions apply to the complete content of this application. The word "comprising" does not exclude other elements or steps, and the indefinite article "a" or "an" does not exclude a plurality. A single step may fulfill the functions of several features recited in the claims. The terms "essentially", "about", "approximately" and the like in connection with an attribute or a value particularly also define exactly the attribute or exactly the value, respectively. The term "about" in the context of a given numerate value or range refers to a value or range that is within 20 %, within 10 %, or within 5 % of the given value or range.

"Homology, identity or similarity" refer to the degree of sequence similarity between two polypeptides or between two polynucleotide molecules compared by sequence alignment. The degree of similarity between two discrete polynucleotide sequences being compared is a function of the number of identical, or matching, nucleotides at comparable positions. The degree of similarity expressed in terms of percent identity may be determined by visual inspection and mathematical calculation. Alternatively, the percent identity of two polynucleotide sequences may be determined by comparing sequence information using the GAP computer program, version 6.0 described by Devereux et al. (Nucl. Acids Res. 12:387, 1984) and available from the University of Wisconsin Genetics Computer Group (UWGCG). Typical default parameters for the GAP program include: (1) a unary comparison matrix (comprising a value of 1 for identities and 0 for non-identities) for nucleotides, and the weighted comparison matrix of Gribskov and Burgess, Nucl. Acids Res. 14:6745, 1986, as described by Schwartz and Dayhoff, eds., Atlas of Protein Sequence and Structure, National Biomedical Research Foundation, pp. 353-358, 1979; (2) a penalty of 3.0 for each gap and an additional 0.10 penalty for each symbol in each gap; and (3) no penalty for end gaps. Various programs known to persons skilled in the art of sequence comparison can be alternatively utilized.

The term "upstream" refers to a relative direction/position with respect to a reference element along a linear polynucleotide sequence, which indicates a direction/ position towards the 5' end of the polynucleotide sequence. "Upstream" may be used interchangeably with the "5' end of a reference element."

The term "downstream" refers to a relative direction/position with respect to a reference element along a linear polynucleotide sequence, which indicates a direction/ position towards the 3' end of the polynucleotide sequence. "Downstream" may be used interchangeably with the "3' end of a reference element."

"Fragments" or "truncations" (eg. truncated proteins) include any portion of an amino acid sequence of a polypeptide which retains at least one structural or functional characteristic of the subject post-translational enzyme or polypeptide.

A "fusion protein" includes a protein in which a peptide sequence from a different protein is covalently linked together by one or more peptide bonds. A "fusion protein partner" refers to that portion of the fusion protein which induces the formation of a protein body in a plant.

The term "operably linked" refers to the joining of distinct DNA elements, fragments, or sequences to produce a functional transcriptional unit. Suitably, therefore, a regulatory sequence that regulates the transcription of said DNA elements, fragments, or sequences is positioned upstrea m thereof.

The terms "purify" and "isolate" and grammatical variations thereof, are used to mean the separation or removal, whether completely or partially, of at least one impurity from a mixture, which thereby improves the level of purity of MIS in the composition.

"Transformation" refers to the alteration of genetic material of a cell resulting from the introduction of exogenous genetic material into the cell. A number of methods are available in the art for transforming a plant cell which are all encompassed herein, including biolistics, gene gun techniques, Agrobacterium-mediated transformation, viral vector-mediated transformation and electroporation. A transgenic plant can be made by regenerating plant cells that have been genetically transformed.

"Agroinfiltration" or "infiltration" is a method for inducing transient expression of genes in a plant or to produce a desired protein. In one aspect, the technique involves injecting a suspension of Agrobacterium cells into the underside of a plant leaf, where it transfers the desired gene to plant cells. Vacuum infiltration is another method for introducing large numbers of Agrobacterium cells into plant tissue. In this procedure, leaf disks, leaves, or whole plants are submerged in a container with the suspension, and the container is placed in a vacuum chamber. The vacuum is then applied which causes air to exit through the stomata. When the vacuum is released, the pressure difference forces the suspension through the stomata and into the plant tissue.

The term "plant" refers to any plant at any stage of its life cycle or development, and its progenies. The plant may be or may be derived from a naturally occurring, mutant, non-naturally occurring or transgenic plant.

The term "plant cell" refers to a structural and physiological unit of a plant. The plant cell may be in form of a protoplast without a cell wall, an isolated single cell, a cultured cell, a clump of two or more cells or as a part of higher organized unit such as but not limited to, plant tissue, a plant organ, or a whole plant.

The term "plant material" refers to any solid, or liquid composition, or a combination thereof, obtained or obtainable from a plant, including leaves, stems, roots, flowers or flower parts, fruits, pollen, egg cells, zygotes, seeds, cuttings, secretions, extracts, cell or tissue cultures, or any other parts or products of a plant. In one embodiment, the plant material is or is derived from a leaf - such as a green leaf.

### DETAILED DESCRIPTION

The method for producing MIS in a plant comprises, in one aspect, incubating a plant into which has been introduced a nucleic acid construct comprising, consisting or consisting essentially of a nucleic acid sequence encoding a protein that induces the formation of a protein body in a plant; and a nucleic acid sequence encoding MIS, wherein said nucleic acid sequences are operably linked to each other.

The nucleic acid sequence encoding Mullerian Inhibitor encompasses nucleic acid sequences with substantial homology (that is, sequence similarity) or substantial identity to the nucleic acid sequence of MIS, preferably human MIS. MIS is encoded in humans by the AMH gene (Cell (1986) 45 (5): 685-98). In humans, the gene for MIS is located on chromosome 19p13.3 and has Genbank accession number NG_012190. The term MIS also encompasses pre-pro-MIS, pro-MIS, and fragments of MIS - such as the C-terminal fragment (also referred to as mature MIS), or polypeptide with substantial homology (that is, sequence similarity) or substantial identity thereto. As described herein, a variant of a MIS polynucleotide may have at least 60%, 65%, 70%, 71%, 72%, 73%, 74%, 75%, 80%, 85%, 90%, 95%, 96%, 97%, 98%, 99% or 100% sequence identity to the sequence reported in Genbank accession number NG_012190. The MIS variant may be a variant of MIS displaying the biological and/or immunological activity of an MIS protein. As used herein, the phrase "biological activity of an MIS protein" means that the MIS-like polypeptide has at least one biological activity which is substantially the same as or is similar to at least one naturally occurring MIS protein - such as the ability to stimulate regression of the Mullerian ducts or is cytotoxic to one or more types of ovarian tumour cells, for example, the cell line HOC-21, and preferably, it both stimulates regression of the Mullerian ducts and is cytotoxic to one or more types of ovarian tumour cells. As used herein, the phrase "immunological activity of an MIS protein" refers to the ability of an MIS-like polypeptide to cross-react with an antibody which is specific for a naturally occurring MIS protein. An example of such an antibody is disclosed in US4,487,833. Variants of MIS may include amino acids in addition to those of a native MIS protein or it may not include all of the amino acids of native MIS protein. The variant may have deletions, insertions or substitutions of amino acid residues, which produce a silent change and result in a functionally equivalent protein. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine. Conservative substitutions may be made, for example according to the Table below. Amino acids in the same block in the second column and preferably in the same line in the third column may be substituted for each other:

| | | |
|---|---|---|
| ALIPHATIC | Non-polar | Gly Ala Pro Ile Leu Val |
| | Polar - uncharged | Cys Ser Thr Met Asn Gly |
| | Polar - charged | Asp Glu Lys Arg |
| AROMATIC | | His Phe TrpTyr |

Also, this polypeptide may be a mature protein or an immature protein or a protein derived from an immature protein (for example, a protein wherein only a portion of the signal sequence has been cleaved). Examples of such polypeptides are derivatives of MIS polypeptides which have been prepared by modification of the MIS amino acid sequence to achieve an improvement in properties, e.g., greater storage stability or increased half-life *in vivo.*

The methods may also be used to produce the C-terminal fragment of MIS ("mature MIS") which includes proteins corresponding to or structurally similar to the about 12.5 kDa (about 25 kDa under non-reducing conditions) C-terminal fragment of MIS resulting from proteolytic cleavage at residue 427 of the intact 535 amino acid human MIS monomer. The proteolytic cleavage recognition site is at residue 443 of the 551 amino acid bovine MIS molecule. In particular, this fragment includes the about 25 kDa homodimeric C-terminal fragment of MIS. Mullerian duct regression and antiproliferative activities reside in the C-terminal domain of MIS and so for some applications only this portion of MIS may be required. The methods may also be used to express the N-terminal fragment of MIS which includes the about 57 kDa fragment resulting from cleavage at residue 427 of the intact 535 amino acid human MIS monomer (residue 443 of the 551 amino acid bovine MIS).

The invention also encompasses the use of a nucleic acid sequence that encodes MIS, or a fragment thereof, including but not limited to the C-termainal fragment of MIS. The nucleic acid sequence may not be identical to the naturally occurring MIS so long as it encodes MIS or a variant of MIS. Accordingly, the nucleic acid sequence may have at least 80%, 85%, 90%, 95%, 96%, 97%, 98%, 99% or 100% sequence identity relative to the naturally occurring coding sequence of MIS. In a preferred embodiment, the nucleic acid sequence encoding MIS used in the invention has been optimized for expression in plants by substituting certain codons with alternative codons in accordance with the codon usage in plants.

In one embodiment, the nucleic acid sequence of MIS is a coding sequence which has been optimised for expression in plants and comprises, consists or consists essentially the sequence set forth in SEQ ID No. 1 or is a variant, fragment or homologue thereof. In one embodiment, the amino acid sequence of MIS comprises, consists or consists essentially the sequence set forth in SEQ ID No. 2 or is a variant, fragment or homologue thereof.

A nucleic acid sequence encoding a protein that induces the formation of a protein body in a plant is also included. Protein bodies are naturally-occurring structures in certain plant seeds that have evolved to concentrate storage proteins intracellularly in eukaryotic cells while retaining correct folding and biological activity. Protein bodies share some of the characteristics of the inclusion bodies from bacteria. They are dense, and contain a high quantity of aggregated proteins that are tightly packed by hydrophobic interactions. The storage proteins can be inserted into the lumen of the endoplasmic reticulum via a signal peptide and are assembled either in the endoplasmic reticulum developing specific organelles called endoplasmic reticulum-derived protein bodies or in protein storage vacuoles. Examples of proteins that induce the formation of a protein body in a plant include storage proteins or modified storage proteins - such as prolamins or modified prolamins or prolamin domains. Gamma-zein is a maize storage protein and is one of the four maize prolamins. As other cereal prolamins, alpha- and gamma-zeins are biosynthesized in membrane-bound polysomes at the cytoplasmic side of the rough endoplasmic reticulum, assembled within the lumen and then sequestered into endoplasmic reticulum-derived protein bodies.

Suitable prolamins include, but are not limited to, gamma-zein, alpha gliadin, the rice rP13 protein and the 22 kDa N-terminal fragment of the maize alpha-zein.

In a preferred embodiment, the protein that induces the formation of a protein body in a plant is gamma-zein, which is composed of four characteristic domains i) a peptide signal of 19 amino acids, ii) the repeat domain comprising eight units of the hexapeptide PPPVHL (53 aa), iii) the ProX domain where proline residues alternate with other amino acids (29 aa) and iv) the hydrophobic cysteine rich C-terminal domain.

One or more non-naturally occurring repeat sequence motifs can be incorporated or substituted into gamma-zein which may improve the expression level of MIS in a plant cell. Where the non-naturally occurring repeat sequence motif(s) are substituted, the repeat domain or the ProX domain or both, of these domains are mutated to create the non-naturally occurring sequence motif. Since the repeat sequence is a non-naturally occurring sequence motif then it will not be present in the native gamma-zein (for example, native maize gamma zein) sequence. In one embodiment, the non-naturally occurring repeat sequence motif(s) are incorporated or substituted into the repeat domain of gamma-zein. In another embodiment, the non-naturally occurring repeat sequence motif(s) are incorporated into the ProX domain of gamma-zein. In another embodiment, the non-naturally occurring repeat sequence motif(s) are incorporated into the repeat domain and the ProX domain of gamma-zein In a preferred embodiment, the non-naturally occurring repeat sequence motif(s) are substituted into a fragment which consists essentially of the repeat domain and the ProX domain of gamma-zein. An example of such a fragment comprises, consists or consists essentially of at least amino acid residues 22 to 109, 22 to 110, 22 to 111, 22 to 112, 22 to 113, 22 to 114 or 22 to 115 of gamma-zein. In other words, the N-terminus of the fusion protein partner comprises, including the signal peptide of gamma-zein, the first 105 to 115 amino acids of gamma-zein with various substitutions as described in the foregoing.

Non-limiting examples of the non-naturally occurring repeat sequence motifs are selected from the group consisting of: (PPPVAL)n or (Pro Pro Pro Val Ala Leu)n; (PPPVEL)n or (Pro Pro Pro Val Glu Leu)n; (PPPAPA)n or (Pro Pro Pro Ala Pro Ala)n; and (PPPEPE)n or (Pro Pro Pro Glu Pro Glu)n or a combination of two or more thereof, wherein n = 1 to 5, 1 to 6, 1 to 7, 1 to 8, 1 to 9, 1 to 10, 1 to 15, 1 to 20 or 1 to 25 and so on. In a preferred embodiment, n = 7 or 8.

Beside, alanine and glutamate, other amino acids (such as but not limited to threonine) can also be used in the proline-rich non-naturally repeat sequence, (for example, (PPPVTL).

In another embodiment, combinations of two or more of different non-naturally occurring repeat sequence motifs can be used in the repeat domain, the ProX domain or both - such as (PPPVAL)n and (PPPVEL)n; or (PPPAPA)n and (PPPEPE)n; or (PPPVAL)n and (PPPVEL)n and (PPPAPA)n; or (PPPVEL)n and (PPPAPA)n and (PPPEPE)n; or (PPPVAL)n and (PPPVEL)n and (PPPAPA)n and (PPPEPE)n.

In one embodiment, the (PPPAPA)n sequence in the repeat domain of gamma zein comprises, consists or consists essentially of the sequence set forth in SEQ ID No. 6.

In one embodiment, the (PPPEPE)n sequence in the repeat domain of gamma zein comprises, consists or consists essentially of the sequence set forth in SEQ ID No. 7.

In one embodiment, the (PPPVEL)n sequence in the repeat domain of gamma zein comprises, consists or consists essentially of the sequence set forth in SEQ ID No. 8.

In one embodiment, the (PPPVAL)n sequence in the repeat domain of gamma zein comprises, consists or consists essentially of the sequence set forth in SEQ ID No. 9.

In one embodiment, the (PPPVTL)n sequence in the repeat domain of gamma zein comprises, consists or consists essentially of the sequence set forth in SEQ ID No. 10.

In one embodiment, the (PPPAPA)n sequence in the ProX domain of gamma-zein comprises, consists or consists essentially of the sequence set forth in SEQ ID No. 11.

In one embodiment, the (PPPEPE)n sequence in the ProX domain of gamma-zein comprises, consists or consists essentially of the sequence set forth in SEQ ID No. 12.

The non-naturally occurring repeat sequence motif(s) may be positioned at the 5' or the 3'-end of the repeat domain and/or the ProX domain of gamma-zein. The non-naturally occurring repeat sequence motif(s) may be positioned at the 5' and the 3'-end of the repeat domain and/or the ProX domain of gamma-zein. In a suitable embodiment, the non-naturally occurring repeat sequence motif(s) is positioned within the repeat domain and/or the ProX domain of gamma-zein. Suitably, said plant cell does not produce protein bodies in the absence of the nucleic acid encoding the fusion protein.

Suitably, the protein body-inducing sequence further includes a sequence encoding a peptide that directs the fusion protein towards the endoplasmic reticulum of a plant cell. The peptide is often referred to as a leader sequence or signal peptide and can be from the same plant in which the fusion protein is expressed or a different plant. Examples of signal peptides are the 19 residue gamma-zein signal peptide sequence (see WO 2004003207); the 19 residue signal peptide sequence of alpha-gliadin or the 21 residue gamma-gliadin signal peptide sequence (see, for example, Plant Cell (1993) 5:443-450 and EMBO J (1984) 3 (6), 1409-11415). Similarly functioning signal peptides from other plants are also reported in the literature. The signal peptide may be a signal peptide that is native to gamma zein and/or MIS. The nucleic acid encoding the signal peptide may be positioned in a nucleic acid construct such that it is expressed at the N- or the C-terminus of the protein. In one embodiment, the signal peptide is expressed at the N-terminus.

Variants with substantial homology (that is, sequence similarity) or substantial identity to gamma-zein are also encompassed herein. A variant of a gamma-zein polynucleotide may have at least 60%, 65%, 70%, 71%, 72%, 73%, 74%, 75%, 80%, 85%, 90%, 95%, 96%, 97%, 98%, 99% or 100% sequence identity to the sequence reported in Genbank accession number NM_001111884. This term also encompasses fragments of gamma-zein with substantial homology (that is, sequence similarity) or substantial identity thereto. The gamma-zein variant may be a variant displaying the biological and/or immunological activity of gamma-zein. As used herein, the phrase "biological activity of gamma-zein" means that the gamma-zein variant has at least one biological activity which is substantially the same as or is similar to naturally occurring gamma-zein. As used herein, the phrase "immunological activity of gamma-zein " refers to the ability of a gamma-zein variant to cross-react with an antibody which is specific for a naturally occurring gamma-zein. Variants of gamma-zein may include amino acids in addition to those of a native gamma-zein protein or it may not include all of the amino acids of native gamma-zein protein. The variant may have deletions, insertions or substitutions of amino acid residues, which produce a silent change and result in a functionally equivalent protein. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine. Conservative substitutions may be made, for example according to the Table below. Amino acids in the same block in the second column and preferably in the same line in the third column may be substituted for each other:

| | | |
|---|---|---|
| ALIPHATIC | Non-polar | Gly Ala Pro |
| | | Ile Leu Val |
| | Polar - uncharged | Cys Ser Thr Met |
| | | Asn Gly |
| | Polar - charged | Asp Glu |
| | | Lys Arg |
| AROMATIC | | His Phe TrpTyr |

The gamma-zein may be a fragment of gamma-zein protein, said fragment comprising a nucleotide sequence that encodes a protein that induces the formation of a protein body in a plant. Thus, by way of example, gamma-zein may encode all or part of the repetition domain of the protein gamma-zein or all or part of the ProX domain.

In one embodiment, the nucleic acid sequence of gamma zein comprises the sequence set forth in SEQ ID No. 3 or is a variant, fragment or homologue thereof.

In one embodiment, the amino acid sequence of gamma zein comprises the sequence set forth in SEQ ID No. 4 or is a variant, fragment or homologue thereof.

In another embodiment, the amino acid sequence of a fragment of gamma zein comprises the sequence set forth in SEQ ID No. 5 or is a variant, fragment or homologue thereof.

Suitably, the nucleic acid sequences are operably linked to each other such that a fusion protein comprising MIS and gamma-zein is expressed in a plant cell. In one embodiment, the nucleic acid molecule comprises MIS at the 5' end and gamma-zein at the 3' end. In another embodiment, the nucleic acid molecule comprises MIS at the 3' end and gamma-zein at the 5' end.

Suitably, the nucleic acid molecule includes a linker sequence between the nucleic acid sequence that induces the formation of a protein body in a plant and the nucleic acid sequence encoding MIS. Said linker sequence may be operably linked thereto. In one embodiment, the linker sequence encodes an amino acid linker in which one or more peptide bonds therein can be specifically cleaved. It may therefore function as a recognition site for an enzyme or an intein and the like such that the two proteins can be separated from each other. The linker can be cleaved by any entity which can specifically cleave one or more peptide bonds. Advantageously, the linker allows for the separation of MIS from the fusion protein which allows MIS to be subsequently purified if desired to thereby provide a substantially homogeneous recombinant MIS protein. Suitably, MIS is not internally cleaved and so fragments of MIS are not created. Suitably, MIS is cleaved such that less than three, two or one residual amino acids remain at the N-terminus or the C-terminus of MIS. Suitably, MIS is cleaved without leaving residual amino acids at the N-terminus or the C-terminus of MIS. In one embodiment, the fusion protein is not cleaved with enterokinase since this cleaves MIS internally.

A preferred method of cleaving the fusion protein to release MIS is to design the fusion protein in such a way that the N-terminus of the fusion partner is linked to the C-terminus of MIS via an amino acid linker in which a peptide bond therein can be specifically cleaved and wherein the amino acid linker does not occur elsewhere in the fusion protein. This approach has the advantage that the cleavage means can by chosen by reference to a specific amino acid sequence - such as a specific recognition sequence. The linker may contain more than the absolute minimum sequence necessary to direct specific cleavage of one or more peptides bonds. The linker may be generated as a result of the union between two nucleic acid sequences. In this embodiment, each sequence contains a number of nucleotides which can become ligated to form a cleavable linker - such as a cleavable recognition site.

A protease may be used to specifically cleave one or more peptide bonds in the linker. The protease can be Ala-C endoprotease. In another embodiment, the protease is Glu-C endoprotease, also known as *Staphylococcus aureus* Protease V8. This protease is a serine protease which selectively cleaves peptide bonds C-terminal to glutamic acid residues. The protease also cleaves at aspartic acid residues at a rate 100-300 times slower than at glutamic acid residues.

In another embodiment, the protease is TEV protease. TEV protease is a highly site-specific cysteine protease that is found in the Tobacco Etch Virus. The optimum cleavage recognition site for this protease is the sequence Glu-Asn-Leu-Tyr-Phe-Gln-(Gly/Ser) and cleavage occurs between the Gln and Gly/Ser residues.

Non-limiting examples of suitable linkers therefore include Glu-Asn-Leu-Tyr-Phe-Gln-(Gly/Ser), (Gly)x, wherein x is 2, 3, 4, 5, 6, 7, 8, 9 or 10 or more or (Gly4Ser)y, wherein y is 2, 3, 4, 5, 6, 7, 8, 9 or 10 or more. In one embodiment, the linker is (Gly)4. In another embodiment the linker is (Gly4Ser)3. In a further embodiment, the sequence encoding MIS is located at the 3'-end of said linker.

According to another embodiment, non-proteolytic means may be used to separate the two proteins. Thus, for example, inteins may be used. A variety of different inteins are known in the art, in which cleavage can be induced under defined conditions - such as reducing conditions. Thus, according to one embodiment, the amino acid linker may encode an intein. The intein may be derived from various bacterial species - such as Synechocystis sp. or Mycobacterium sp. - such as *Mycobacterium xenopi,* for example. The intein may be derived from Saccharomyces sp. - such as *Saccharomyces cerevisiae,* for example, the *Saccharomyces cerevisiae* vacuolar membrane ATPase intein. In one embodiment, the intein is a *Mycobacterium xenopi* Gyrase A intein. Chemicals may also be used to separate MIS from the fusion protein in which case an amino acid linker may not be required.

According to a further embodiment, the nucleic acid construct for use in the method of the present invention comprises: a first nucleic acid sequence encoding a protein that induces the formation of a protein body in a plant, optionally, further comprising a nucleic acid sequence encoding a non-naturally occurring repeat sequence motif; a second nucleic acid sequence encoding MIS; and optionally a third nucleic acid sequence encoding an amino acid linker in which a peptide bond therein can be specifically cleaved; wherein said first, second and third nucleic acid sequences are operably linked to each other.

Various regulatory nucleotide sequences that regulates the transcription of said nucleic acid sequences may therefore also be included. These include transcriptional control elements that are only active in particular cells or tissues at specific times during plant development, such as in vegetative tissues or reproductive tissues. One such example is a promoter which refers to a polynucleotide element/sequence, typically positioned upstream and operably-linked to a double-stranded DNA fragment. A suitable promoter will enable the transcriptional activation of a nucleic acid sequence by recruiting the transcriptional complex, including the RNA polymerase and various factors, to initiate RNA synthesis. Promoters can be derived entirely from regions proximate to a native gene, or can be composed of different elements derived from different native promoters or synthetic DNA segments. According to one embodiment, tissue-specific expression can be used and may be advantageous when expression of one or more polynucleotides in certain tissues is preferred. Examples of tissue-specific promoters under developmental control include promoters that can initiate transcription only (or primarily only) in certain tissues, such as vegetative tissues, for example, roots or leaves, or reproductive tissues, such as fruit, ovules, seeds, pollen, pistols, flowers, or any embryonic tissue. Reproductive tissue-specific promoters may be, for example, anther-specific, ovule-specific, embryo-specific, endosperm-specific, integument-specific, seed and seed coat-specific, pollen-specific, petal-specific, sepal-specific, or combinations thereof. Suitable leaf-specific promoters include pyruvate, orthophosphate dikinase (PPDK) promoter from C4 plant (maize), cab-m1Ca+2 promoter from maize, the Arabidopsis thaliana myb-related gene promoter (Atmyb5), the ribulose biphosphate carboxylase (RBCS) promoters (for example, the tomato RBCS 1, RBCS2 and RBCS3A genes expressed in leaves and light-grown seedlings, RBCS1 and RBCS2 expressed in developing tomato fruits or ribulose bisphosphate carboxylase promoter expressed almost exclusively in mesophyll cells in leaf blades and leaf sheaths at high levels). Suitable senescence-specific promoters include a tomato promoter active during fruit ripening, senescence and abscission of leaves, a maize promoter of gene encoding a cysteine protease. Suitable anther-specific promoters can be used. Suitable root-preferred promoters known to persons skilled in the art may be selected. Suitable seed-preferred promoters include both seed-specific promoters (those promoters active during seed development such as promoters of seed storage proteins) and seed-germinating promoters (those promoters active during seed germination). Such seed-preferred promoters include, but are not limited to, Cim1 (cytokinin-induced message); milps (myo-inositol-1-phosphate synthase); mZE40-2, also known as Zm-40; nuclc; and celA (cellulose synthase). Glob-1 is an embryo-specific promoter. For dicots, seed-specific promoters include, but are not limited to, bean beta-phaseolin, napin, beta-conglycinin, soybean lectin, cruciferin, and the like. For monocots, seed-specific promoters include, but are not limited to, a maize 15 kDa zein promoter, a 22 kDa zein promoter, a 27 kDa zein promoter, a g-zein promoter, a 27 kDa gamma-zein promoter (such as gzw64A promoter, see Genbank Accession #S78780), a waxy promoter, a shrunken 1 promoter, a shrunken 2 promoter, a globulin 1 promoter (see Genbank Accession # L22344), an ltp2 promoter, cim1 promoter, maize end1 and end2 promoters, nuc1 promoter, Zm40 promoter, eep1 and eep2; lec1, thioredoxin H promoter; mlip15 promoter, PCNA2 promoter; and the shrunken-2 promoter. Examples of inducible promoters include promoters responsive to pathogen attack, anaerobic conditions, elevated temperature, light, drought, cold temperature, or high salt concentration. Pathogen-inducible promoters include those from pathogenesis-related proteins (PR proteins), which are induced following infection by a pathogen (for example, PR proteins, SAR proteins, beta-1 ,3-glucanase, chitinase).

In addition to plant promoters, other suitable promoters may be derived from bacterial origin for example, the octopine synthase promoter, the nopaline synthase promoter and other promoters derived from Ti plasmids), or may be derived from viral promoters (for example, 35S and 19S RNA promoters of cauliflower mosaic virus (CaMV), constitutive promoters of tobacco mosaic virus, cauliflower mosaic virus (CaMV) 19S and 35S promoters, or figwort mosaic virus 35S promoter). The regulatory sequence may also contain a transcription termination sequence that is functional in a plant. The regulatory sequence may also contain a translation enhancer functional in plant. An enhancer is a nucleic acid sequence that can recruit transcriptional regulatory proteins such as transcriptional activators, to enhance transcriptional activation by increasing promoter activity. Suitable enhancers can be derived from regions proximate to a native promoter of interest (homologous sources) or can be derived from non-native contexts (heterologous sources) and operably-linked to any promoter of interest to enhance the activity or the tissue-specificity of a promoter. Some enhancers can operate in any orientation with respect to the orientation of a transcription unit. For example, enhancers may be positioned upstream or downstream of a transcriptional unit comprising a promoter and a nucleic acid construct.

The nucleic acid sequence, nucleic acid construct, or vector and the like comprises, in a further embodiment, a nucleic acid sequence encoding a suppressor of gene silencing of, for example, viral origin. In one embodiment, the suppressor of gene silencing is that of a virus selected from the group consisting of Havel river virus (HaRV), Pear latent virus (PeLV), Lisianthus necrosis virus, Grapevine Algerian latent virus, Pelargonium necrotic spot virus (PeNSV), Cymbidium ringspot virus (CymRSV), Artichoke mottled crinkle virus (AMCV), Carnation Italian ringspot virus (CIRV), Lettuce necrotic stunt virus, rice yellow mottle virus (RYMV), potato virus X (PVX), African cassava mosaic virus (ACMV), Cucumber mosaic virus (CMV), Cucumber necrosis virus (CNV), potato virus Y (PVY), tobacco etch virus (TEV), and Tomato bushy stunt virus (TBSV) or a combination of two or more thereof. Examples of suppressor of gene silencing that can be used in the invention include but are not limited to the p1 protein of rice yellow mottle virus (RYMV), the p25 protein of potato virus X (PVX), the AC2 protein of African cassava mosaic virus (ACMV), the 2b protein of cucumber mosaic virus (CMV), the 19 kDa p19 protein of Cucumber necrosis virus (CNV), the helper-component proteinase (HcPro) of potato virus Y (PVY), tobacco etch virus (TEV) and Tomato bushy stunt virus (TBSV) In one embodiment, the suppressor of gene silencing is HcPro of tobacco etch virus (TEV). In another embodiment, the suppressor of gene silencing is the p19 protein of Tomato bushy stunt virus (TBSV). Accordingly, in a further embodiment, there is provided a nucleic acid construct comprising: a first nucleic acid sequence encoding a protein that induces the formation of a protein body in a plant, optionally, further comprising a nucleic acid sequence encoding a non-naturally occurring repeat sequence motif; a second nucleic acid sequence encoding MIS; and optionally a third nucleic acid sequence encoding an amino acid linker in which a peptide bond therein can be specifically cleaved; wherein said first, second and third nucleic acid sequences are operably linked to each other and optionally, a regulatory nucleotide sequence that regulates the transcription of said nucleic acid sequence(s) and optionally an expressible nucleic acid encoding a suppressor of gene silencing, suitably of viral origin. In an alternative embodiment, the expressible nucleic acid encoding a suppressor of gene silencing can be a separate second nucleic acid molecule or a part of a second nucleic acid which is introduced to the plant or plant cells, and coexpressed in the plant or plant cells that are also producing MIS.

The plant host cell used for the expression of recombinant MIS may be derived or derivable from a plant or it may be a cultured plant cell that is cultured outside of a plant. Thus, in one embodiment, the plant is a plant cell - such as a plant cell grown in culture or outside of a plant such as an in vitro grown plant cell or clumps of cells. Non-limiting examples of plants include monocots and dicots, such as crop plants, ornamental plants, and non-domesticated or wild plants. Further examples include plants of commercial or agricultural interest, such as crop plants (especially crop plants used for human food or animal feed), wood- or pulp-producing trees, vegetable plants, fruit plants, and ornamental plants.

Techniques for introducing (for example, transforming or infiltrating) one or more nucleic acid molecules into a plant - such as monocotyledonous and dicotyledonous plants - are known in the art. Any method may be used to introduce the nucleic acid molecule(s), vectors, constructs and the like into a plant. By way of example, they may be introduced into a plant by biolistics or gene gun techniques employing microparticles coated with the construct(s) Agrobacterium-mediated transformation (for example, using *A*. *radiobacter, A. rhizogenes, A. rubi,* or A. *tumefaciens),* viral vector-mediated transformation, electroporation and infiltration by Agrobacterium cells, also referred to as agroinfiltration. In one embodiment, Agrobacterium-mediated transformation of plant cells is used. In another embodiment, agroinfiltration is used to introduced the nucleic acids into a whole plant, an intact plant, or a part thereof. Agroinfiltration can be carried out under reduced air pressure or a vacuum by techniques and apparatus known in the art.

The introduction of a nucleic acid into a plant may give rise to stable expression of the protein encoded by the nucleic acid. Typically, stable expression will result in the integration of the nucleic acid into the host genome so as to create a transgenic plant and the nucleic acid will be passed onto the next generation. The introduction of a nucleic acid into a plant may give rise to transient expression of the protein encoded by the nucleic acid. Transient expression does not necessarily rely on the integration of the nucleic acid into the host genome. Typically, tobacco plants infiltrated with Agrobacterium cells are incubated for 5, 10, 15, or 20 days or more before the plant parts are harvested to recover the recombinantly produced protein. Both forms of expression are contemplated by the present invention.

The plants into which the nucleic acid has been introduced can be incubated and progeny obtained optionally under selection if a selectable marker gene is employed,. These progeny may be used to prepare transgenic seeds, or alternatively, bred with another plant. The seeds obtained from such progeny may be germinated, cultivated, and used to prepare subsequent generations of offspring which comprise the nucleic acid originally introduced. An immature embryo or embryogenic calli from a plant may be used as a starting material. These techniques are routine and well known to one of ordinary skill in the art. Once the plant matures then the tissue into which the nucleotide sequence is expressed is harvested and recovered therefrom using the methods described herein.

For example, stable plant transformation can be carried out as follows: vectors are transferred into *Agrobacterium tumefaciens.* Tobacco *(Nicotiana benthamiana or N. tabacum)* leaf discs are transformed according to the method of Draper et al. (1988) In: Plant Genetic Transformation and Gene Expression. A Laboratory Manual (Eds. Draper, J. , Scott, R., Armitage, P. and Walden, R.), Blackwell Scientific Publications. Regenerated plants are selected on medium comprising 200 mg/L kanamycin and transferred to a greenhouse. Transformed tobacco plants having the highest transgene product levels are cultivated to obtain a T1 generation. Developing leaves (approximately 12 cm long) are harvested, immediately frozen with liquid nitrogen and stored at-80°C for further experiments.

The plant host cell may be a grain crop plants (such as wheat, oat, barley, maize, rye, triticale, rice, millet, sorghum, quinoa, amaranth, and buckwheat); forage crop plants (such as forage grasses and forage dicots including alfalfa, vetch, clover, and the like); oilseed crop plants (such as cotton, safflower, sunflower, soybean, canola, rapeseed, flax, peanuts, and oil palm); tree nuts (such as walnut, cashew, hazelnut, pecan, almond, and the like); sugarcane, coconut, date palm, olive, sugarbeet, tea, and coffee; wood- or pulp-producing trees; vegetable crop plants such as legumes (for example, beans, peas, lentils, alfalfa, peanut), lettuce, asparagus, artichoke, celery, carrot, radish, the brassicas (for example, cabbages, kales, mustards, and other leafy brassicas, broccoli, cauliflower, Brussels sprouts, turnip, kohlrabi), cucurbits (for example, cucumbers, melons, summer squashes, winter squashes), alliums (for example, onions, garlic, leeks, shallots, chives), members of the Solanaceae (for example, tomatoes, eggplants, potatoes, peppers, groundcherries), and members of the Chenopodiaceae (for example, beet, chard, spinach, quinoa, amaranth); fruit crop plants such as apple, pear, citrus fruits (for example, orange, lime, lemon, grapefruit, and others), stone fruits (for example, apricot, peach, plum, nectarine), banana, pineapple, grape, kiwifruit, papaya, avocado, and berries; and ornamental plants including ornamental flowering plants, ornamental trees and shrubs, ornamental groundcovers, and ornamental grasses. Further examples of dicot plants include, but are not limited to, canola, cotton, potato, quinoa, amaranth, buckwheat, safflower, soybean, sugarbeet, and sunflower, more suitably soybean, canola, and cotton. Further examples of monocots include, but are not limited to, wheat, oat, barley, maize, rye, triticale, rice, ornamental and forage grasses, sorghum, millet, and sugarcane.

The plant host cell may be or may be derived from a monocotyledonous or dicotyledonous plant or a plant cell system, including species from one of the following families: Acanthaceae, Alliaceae, Alstroemeriaceae, Amaryllidaceae, Apocynaceae, Arecaceae, Asteraceae, Berberidaceae, Bixaceae, Brassicaceae, Bromeliaceae, Cannabaceae, Caryophyllaceae, Cephalotaxaceae, Chenopodiaceae, Colchicaceae, Cucurbitaceae, Dioscoreaceae, Ephedraceae, Erythroxylaceae, Euphorbiaceae, Fabaceae, Lamiaceae, Linaceae, Lycopodiaceae, Malvaceae, Melanthiaceae, Musaceae, Myrtaceae, Nyssaceae, Papaveraceae, Pinaceae, Plantaginaceae, Poaceae, Rosaceae, Rubiaceae, Salicaceae, Sapindaceae, Solanaceae, Taxaceae, Theaceae, or Vitaceae.

Suitable species may include members of the genera Abelmoschus, Abies, Acer, Agrostis, Allium, Alstroemeria, Ananas, Andrographis, Andropogon, Artemisia, Arundo, Atropa, Berberis, Beta, Bixa, Brassica, Calendula, Camellia, Camptotheca, Cannabis, Capsicum, Carthamus, Catharanthus, Cephalotaxus, Chrysanthemum, Cinchona, Citrullus, Coffea, Colchicum, Coleus, Cucumis, Cucurbita, Cynodon, Datura, Dianthus, Digitalis, Dioscorea, Elaeis, Ephedra, Erianthus, Erythroxylum, Eucalyptus, Festuca, Fragaria, Galanthus, Glycine, Gossypium, Helianthus, Hevea, Hordeum, Hyoscyamus, Jatropha, Lactuca, Linum, Lolium, Lupinus, Lycopersicon, Lycopodium, Manihot, Medicago, Mentha, Miscanthus, Musa, Nicotiana, Oryza, Panicum, Papaver, Parthenium, Pennisetum, Petunia, Phalaris, Phleum, Pinus, Poa, Poinsettia, Populus, Rauwolfia, Ricinus, Rosa, Saccharum, Salix, Sanguinaria, Scopolia, Secale, Solanum, Sorghum, Spartina, Spinacea, Tanacetum, Taxus, Theobroma, Triticosecale, Triticum, Uniola, Veratrum, Vinca, Vitis, and Zea.

Other suitable species may include Panicum spp., Sorghum spp., Miscanthus spp., Saccharum spp., Erianthus spp., Populus spp., Andropogon gerardii (big bluestem), Pennisetum purpureum (elephant grass), Phalaris arundinacea (reed canarygrass), Cynodon dactylon (bermudagrass), Festuca arundinacea (tall fescue), Spartina pectinata (prairie cord-grass), Medicago sativa (alfalfa), Arundo donax (giant reed), Secale cereale (rye), Salix spp. (willow), Eucalyptus spp. (eucalyptus), Triticosecale (triticum--wheat.times.rye), bamboo, Helianthus annuus (sunflower), Carthamus tinctorius (safflower), Jatropha curcas (jatropha), Ricinus communis (castor), Elaeis guineensis (palm), Linum usitatissimum (flax), Brassica juncea, Beta vulgaris (sugarbeet), Manihot esculenta (cassaya), Lycopersicon esculentum (tomato), Lactuca sativa (lettuce), Musa paradisiaca (banana), Solanum tuberosum (potato), Brassica oleracea (broccoli, cauliflower, Brussels sprouts), Camellia sinensis (tea), Fragaria ananassa (strawberry), Theobroma cacao (cocoa), Coffea arabica (coffee), Vitis vinifera (grape), Ananas comosus (pineapple), Capsicum annum (hot & sweet pepper), Allium cepa (onion), Cucumis melo (melon), Cucumis sativus (cucumber), Cucurbita maxima (squash), Cucurbita moschata (squash), Spinacea oleracea (spinach), Citrullus lanatus (watermelon), Abelmoschus esculentus (okra), Solanum melongena (eggplant), Rosa spp. (rose), Dianthus caryophyllus (carnation), Petunia spp. (petunia), Poinsettia pulcherrima (poinsettia), Lupinus albus (lupin), Uniola paniculata (oats), bentgrass (Agrostis spp.), Populus tremuloides (aspen), Pinus spp. (pine), Abies spp. (fir), Acer spp. (maple), Hordeum vulgare (barley), Poa pratensis (bluegrass), Lolium spp. (ryegrass) and Phleum pratense (timothy), Panicum virgatum (switchgrass), Sorghum bicolor (sorghum, sudangrass), Miscanthus giganteus (miscanthus), Saccharum sp. (energycane), Populus balsamifera (poplar), Zea mays (corn), Glycine max (soybean), Brassica napus (canola), Triticum aestivum (wheat), Gossypium hirsutum (cotton), Oryza sativa (rice), Helianthus annuus (sunflower), Medicago sativa (alfalfa), Beta vulgaris (sugarbeet), or Pennisetum glaucum (pearl millet).

In a particularly suitable embodiment, the plant host cell may be or may be derived from a naturally occurring, a mutant, a non-naturally occurring or a transgenic tobacco plant. A tobacco plant includes plants of the genus *Nicotiana,* various species of *Nicotiana,* including *N*. *rustica* and *N. tabacum.* Other species include *N. acaulis, N. acuminata, N. acuminata var. multiflora, N. africana, N. alata, N. amplexicaulis, N. arentsii, N. attenuata, N. benavidesii, N. benthamiana, N. bigelovii, N. bonariensis, N. cavicola, N. clevelandii, N. cordifolia, N. corymbosa, N. debneyi, N. excelsior, N. forgetiana, N. fragrans, N. glauca, N. glutinosa, N. goodspeedii, N. gossei, N. hybrid, N. ingulba, N. kawakamii, N. knightiana, N. langsdorffii, N. linearis, N. longiflora, N. maritima, N. megalosiphon, N. miersii, N. noctiflora, N. nudicaulis, N. obtusifolia, N. occidentalis, N. occidentalis subsp. hesperis, N. otophora, N. paniculata, N. pauciflora, N. petunioides, N. plumbaginifolia, N. quadrivalvis, N. raimondii, N. repanda, N. rosulata, N. rosulata subsp. ingulba, N. rotundifolia, N. setchellii, N. simulans, N. solanifolia, N. spegazzinii, N. stocktonii, N. suaveolens, N. sylvestris, N. tabacum. N. thyrsiflora, N. tomentosa, N. tomentosiformis, N. trigonophylla, N. umbratica, N. undulata, N. velutina, N. wigandioides, and N. x sanderae.*

The use of a plant host cell that is or is derived from cultivars or elite cultivars is also contemplated. Non-limiting examples of varieties or cultivars are: BD 64, CC 101, CC 200, CC 27, CC 301, CC 400, CC 500, CC 600, CC 700, CC 800, CC 900, Coker 176, Coker 319, Coker 371 Gold, Coker 48, CD 263, Denzizli, DF911, Galpao tobacco, GL 26H, GL 350, GL 600, GL 737, GL 939, GL 973, HB 04P, K 149, K 326, K 346, K 358, K394, K 399, K 730, KDH 959, KT 200, KT204LC, KY10, KY14, KY 160, KY 17, KY 171, KY 907, KY907LC, KTY14xL8 LC, Karabaglar, Little Crittenden, McNair 373, McNair 944, msKY 14xL8, Narrow Leaf Madole, NC 100, NC 102, NC 2000, NC 291, NC 297, NC 299, NC 3, NC 4, NC 5, NC 6, NC7, NC 606, NC 71, NC 72, NC 810, NC BH 129, NC 2002, Neal Smith Madole, OXFORD 207, 'Perique' tobacco, PVH03, PVH09, PVH19, PVH50, PVH51, R 610, R 630, R 7-11, R 7-12, RG 17, RG 81, RG H51, RGH 4, RGH 51, RS 1410, Speight 168, Speight 172, Speight 179, Speight 210, Speight 220, Speight 225, Speight 227, Speight 234, Speight G-28, Speight G-70, Speight H-6, Speight H20, Speight NF3, TI 1406, TI 1269, TN 86, TN86LC, TN 90, TN 97, TN97LC, TN D94, TN D950, TR (Tom Rosson) Madole, Turkish Samson, VA 309, VA359, DAC, Mata, Fina, PO2, BY-64, AS44, RG17, RG8, HB04P, Basma Xanthi BX 2A, Coker 319, Hicks, McNair 944 (MN 944), Burley 21, K149, Yaka JB 125/3, Kasturi Mawar, NC 297, Coker 371 Gold, PO2, Wislica, Simmaba, Turkish Samsun, AA37-1, B13P, F4 from the cross BU21 x Hoja Parado line 97, Samsun, PO1 LA B21, LN KY171, TI 1406, Basma, Galpao, Beinhart 1000-1, or Petico. Non-limiting examples of N. tabacum cultivars are AA 37-1, B 13P, Xanthi (Mitchell-Mor), KTRD#3 Hybrid 107, Bel-W3, 79-615, Samsun Holmes NN, KTRDC#2 Hybrid 49, KTRDC#4 Hybrid 110, Burley 21, BY-64, KTRDC#5 KY 160 SI, KTRDC#7 FCA, KTRDC#6 TN 86 SI, Coker 371 Gold, K 149, K 326, K 346, K 358, K 394, K 399, K 730, KY 10, KY 14, KY 160, KY 17, KY 8959, KY 9, KY 907, MD 609, McNair 373, NC 2000, PG 01, PG 04, M066, PO1, PO2, PO3, RG 11, RG 17, RG 8, Speight G-28, TN 86, TN 90, VA 509, AS44, Banket A1, Basma Drama B84/31, Basma I Zichna ZP4/B, Basma Xanthi BX 2A, Batek, Besuki Jember, C104, Coker 319, Coker 347, Criollo Misionero, DAC Mata Fina, Delcrest, Djebel 81, DVH 405, Galpão Comum, HB04P, Hicks Broadleaf, Kabakulak Elassona, Kasturi Mawar, Kutsage E1, KY 14xL8, KY 171, LA BU 21, McNair 944, NC 2326, NC 71, NC 297, NC 3, PVH 03, PVH 09, PVH 19, PVH 2110, Red Russian, Samsun, Saplak, Simmaba, Talgar 28, Turkish Samsun, Wislica, Yayaldag, NC 4, TR Madole, Prilep HC-72, Prilep P23, Prilep PB 156/1, Prilep P12-2/1, Yaka JK-48, Yaka JB 125/3, TI-1068, KDH-960, TI-1070, TW136, Samsun NN, Izmir, Basma, TKF 4028, L8, TKF 2002, TN90, GR141, Basma xanthi, GR149, GR153, Petit Havana or Xanthi NN.

The plant host cell may be modified to improve the expression and/or activity of the recombinant MIS protein. The host cell may, for example, be modified to include chaperone proteins that further promote the formation of MIS. The host cell may be modified to include a repressor protein to more efficiently regulate the expression of MIS or even an enhancer protein to improve expression levels.

The method for producing MIS in a plant comprises the second step of growing said plant under conditions that allow for the expression of MIS as a fusion protein in said plant. Accordingly, the MIS polypeptide is prepared by incubating (for example, culturing) the plant cells under culture conditions suitable to express the polypeptide as a fusion protein. The resulting polypeptide is expressed in the endoplasmic reticulum of a plant cell in the form of protein bodies.

MIS expression may be measured by detecting the amount of mRNA encoding an MIS polypeptide in the cell which can be quantified by, for example, PCR or Northern blot. Where a change in the amount of MIS polypeptide in the sample is being measured, detecting MIS by use of anti-MIS antibodies can be used to quantify the amount of MIS polypeptide in the cell using known techniques. Alternatively the biological activity of MIS can be measured as described herein.

Various methods may be utilised to recover the protein bodies comprising the fusion protein. The recombinant protein body-like assemblies have a density that can be predetermined for a particular fusion protein. The predetermined density is typically greater than that of substantially all of the endogenous host cell proteins present in the homogenate, and is typically about 1.1 to about 1.35 g/ml. The high density of the protein bodies may be due to the general ability of the recombinant fusion proteins to assemble as multimers and accumulate. When expressed in plants, the protein bodies are typically spherical in shape with diameters of about 1 micron and have a surrounding membrane.

Recovery of the protein bodies by density is typically carried out using a centrifuge. The centrifugation may be carried out in the presence of a differential density-providing solute - such as a salt (for example, caesium chloride) or a sugar (for example, sucrose). Regions of different density may be formed in the homogenate to provide a region that contains a relatively enhanced concentration of the protein bodies and a region that contains a relatively depleted concentration of the protein bodies. The protein body-depleted region may be separated from the region of relatively enhanced concentration of protein bodies, thereby recovering said fusion protein. The protein bodies can be collected or can be treated with one or more reagents or subjected to one or more procedures prior to isolation of the protein bodies comprising the fusion protein, as described herein. In some embodiments, the collected protein bodies are used as is, without the need to isolate the fusion protein.

In some embodiments, one low speed centrifugation step may be sufficient to recover the protein bodies in the form of a pellet. Thus, by way of example, centrifugation at 200 x g for 10 minutes at 4 °C may be sufficient. In other embodiments, more than one centrifugation step may be performed in which the low speed centrifugation step is combined with one or more higher speed centrifugation steps. Thus, by way of example, a centrifugation step of about 200 x g for 10 minutes at 4 °C to remove solids and cell debris may be combined with a higher speed centrifugation step of, for example, about 6000 x g for 10 minutes at 4 °C to recover the fusion protein in the pellet.

This centrifugation step may optionally be followed by one or wash steps in a solution comprising a surfactant together with a further optional centrifugation step to concentrate and enrich the protein bodies comprising the fusion protein prior to solubilisation thereof. The further optional centrifugation step may be carried out between washes. In one embodiment, the surfactant used is Triton X-100, suitably 1% Triton X-100. In another embodiment, the further centrifugation step is carried out at about 6000 x g for 10 minutes at 4 °C which may occur between washes.

Thus, according to one embodiment of the invention, the method comprises the additional step of: (c) recovering the protein body comprising the fusion protein from the plant or plant material, preferably wherein step (c) comprises the steps of: (i) homogenising the plant material; (ii) centrifuging the homogenised plant material at low speed, preferably, about 200 x g; (iii) centrifuging the homogenised plant material at a higher speed than step (ii), preferably, about 6000 x g; and (iv) recovering the protein bodies comprising the fusion protein in the pelleted fraction.

In order to solubilise the fusion protein various buffers and reagents may be used. By way of example, the fusion protein comprising MIS may be obtained from the collected protein bodies by dissolution of the surrounding membrane in an aqueous buffer comprising a detergent and/or a reducing agent. Examples of reducing agents include 2-mercaptoethanol, thioglycolic acid, thioglycolate salts, dithiothreitol (DTT), sulfite or bisulfite ions. Examples of detergents include sodium dodecyl sulfate (SDS), ionic detergents (for example, deoxycholate and lauroylsarcosine), non-ionic detergents (for example, Tween 20, Nonidet P-40 and octyl glucoside) and zwitterionic detergents (for example, CHAPS). Conditions are chosen so as to not disrupt and unfold the attached MIS protein.

The variables that can be tested in order to identify appropriate solubilisation conditions include pH, salt, detergent, reducing agent, as well as other variables such as ratio of components, time and temperature. In one embodiment, solubilisation can be achieved using a buffer comprising urea, dithiothreitol and tris(2-carboxyethyl)phosphine), suitably at a ratio of protein bodies:buffer of 1:10 (w/v). The protein bodies may be incubated with the buffer overnight at room temperature and/or together with a cell disrupter. Various buffers can be employed depending on the desired pH of the buffer. Non-limiting examples of buffer components that can be used to control the pH range include acet ate, citrate, histidine, phosphate, ammonium buffers such as ammonium acetate, succinate, MES, CHAPS, MOPS, MOPSO, HEPES, Tris, and the like, as well as combinations of these TRIS-malic acid-NaOH, maleate, chloroacetate, formate, benzoate, propionate, pyridine, piperazine, ADA, PIPES, ACES, BES, TES, tricine, bicine, TAPS, ethanolamine, CHES, CAPS, methylamine, piperidine, boric acid, carbonic acid, lactic acid, butaneandioic acid, diethylmalonic acid, glycylglycine, HEPPS, HEPPSO, imidazole, phenol, POPSO, succinate, TAPS, amine-based, benzylamine, trimethyl or dimethyl or ethyl or phenyl amine, ethylenediamine, or mopholine. In one embodiment, the buffer has a pH of about 9. In another embodiment, the buffer is 50mM Tris pH 9 comprising TCEP, DTT and urea. In another embodiment, the buffer is 50mM Tris pH9 comprising about 5mM TCEP, about 50mM DTT and about 4M urea.

Accordingly, the method of the present invention may comprise the further step of: (d) solubilising the pelleted fraction comprising the fusion protein, preferably, wherein said solubilisation step comprises the use of a mixture comprising, consisting or consisting essentially of urea, dithiothreitol and (tris(2-carboxyethyl)phosphine). Optionally, the preparation may be centrifuged prior to the next method step, for example at 20000 x g for 10 minutes.

The separated, solubilised fusion protein that comprises the MIS protein is collected. At this stage, the MIS protein may be used as is. Preferably, the MIS protein is further processed.

Accordingly, in one embodiment, the method comprises the further step of releasing MIS from said fusion protein. The separation of MIS from the fusion protein is described herein.

Following separation of MIS from the fusion protein, in a further embodiment, the method comprises the additional step of: (f) purifying the cleaved/released MIS protein. Thus, in one embodiment, the recombinant MIS thus purified is substantially free of other polypeptides as determined by, for example, SDS-PAGE or ELISA. In another embodiment, purified MIS is considered to be a MIS composition which contains less than 100 ppm host protein and suitably less than 90 ppm, less than 80 ppm, less than 70 ppm, less than 60 ppm, less than 50 ppm, less than 40 ppm, less than 30 ppm, less than 20 ppm, less than 10 ppm, or less than 5 ppm host protein, as determined by, for example, SDS-PAGE or ELISA.. The MIS protein obtained or obtainable according to the present invention can have a specific activity of at least 50%, 60%, or 70%, and most suitably at least 80%, 90%, 95% or 100% that of the native protein that the sequence is derived from.

Protein purification may utilise a "cation exchange resin" which is negatively charged, and which has free cations for exchange with cations in an aqueous solution passed over or through the adsorbent or solid phase. Any negatively charged ligand suitable to form the cation exchange resin can be used, for example, a carboxylate, sulfonate and others as described below. Commercially available cation exchange resins include, but are not limited to, for example, those having a sulfonate based group (for example, MonoS, MiniS, Source 15S and 30S, SP Sepharose Fast Flow™, SP Sepharose High Performance from GE Healthcare, Toyopearl SP-650S and SP-650M from Tosoh, Macro-Prep High S from BioRad, Ceramic HyperD S, Trisacryl M and LS SP and Spherodex LS SP from Pall Technologies); a sulfoethyl based group (for example, Fractogel SE, from EMD, Poros S- 10 and S-20 from Applied Biosystems); a sulphopropyl based group (for example, TSK Gel SP 5PW and SP-5PW-HR from Tosoh, Poros HS-20 and HS 50 from Applied Biosystems); a sulfoisobutyl based group (for example, (Fractogel EMD SO₃^{"} from EMD); a sulfoxyethyl based group (for example, SE52, SE53 and Express-Ion S from Whatman), a carboxymethyl based group (for example, CM Sepharose Fast Flow from GE Healthcare, Hydrocell CM from Biochrom Labs Inc., Macro-Prep CM from BioRad, Ceramic HyperD CM, Trisacryl M CM, Trisacryl LS CM, from Pall Technologies, Matrx Cellufme C500 and C200 from Millipore, CM52, CM32, CM23 and Express -Ion C from Whatman, Toyopearl CM- 650S, CM-650M and CM-650C from Tosoh); sulfonic and carboxylic acid based groups (for example BAKEPVBOND Carboxy-Sulfon from J.T. Baker); a carboxylic acid based group (for example, WP CBX from J.T Baker, DOWEX MAC-3 from Dow Liquid Separations, Amberlite Weak Cation Exchangers, DOWEX Weak Cation Exchanger, and Diaion Weak Cation Exchangers from Sigma-Aldrich and Fractogel EMD COO- from EMD); a sulfonic acid based group (e. g., Hydrocell SP from Biochrom Labs Inc., DOWEX Fine Mesh Strong Acid Cation Resin from Dow Liquid Separations, UNOsphere S, WP Sulfonic from J. T. Baker, Sartobind S membrane from Sartorius, Amberlite Strong Cation Exchangers, DOWEX Strong Cation and Diaion Strong Cation Exchanger from Sigma-Aldrich); and a orthophosphate based group (for example, PI 1 from Whatman).

Protein purification may utilise an "anion exchange resin" which is positively charged, thus having one or more positively charged ligands attached thereto. Any positively charged ligand attached to the adsorbent or solid phase suitable to form the anionic exchange resin can be used, such as quaternary amino groups Commercially available anion exchange resins include DEAE cellulose, Poros PI 20, PI 50, HQ 10, HQ 20, HQ 50, D 50 from Applied Biosystems, Sartobind Q from Sartorius, MonoQ, MiniQ, Source 15Q and 30Q, Q, DEAE and ANX Sepharose Fast Flow, Q Sepharose high Performance, QAE SEPHADEX™ and FAST Q SEPHAROSE™ (GE Healthcare), WP PEI, WP DEAM, WP QUAT from J.T. Baker, Hydrocell DEAE and Hydrocell QA from Biochrom Labs Inc., UNOsphere Q, Macro-Prep DEAE and Macro-Prep High Q from Biorad, Ceramic HyperD Q, ceramic HyperD DEAE, Trisacryl M and LS DEAE, Spherodex LS DEAE, QMA Spherosil LS, QMA Spherosil M and Mustang Q from Pall Technologies, DOWEX Fine Mesh Strong Base Type I and Type II Anion Resins and DOWEX MONOSPHER E 77, weak base anion from Dow Liquid Separations, Intercept Q membrane, Matrex Cellufme A200, A500, Q500, and Q800, from Millipore, Fractogel EMD TMAE, Fractogel EMD DEAE and Fractogel EMD DMAE from EMD, Amberiite weak strong anion exchangers type I and II, DOWEX weak and strong anion exchangers type I and II, Diaion weak and strong anion exchangers type I and II, Duolite from Sigma-Aldrich, TSK gel Q and DEAE 5PW and 5PW-HR, Toyopearl SuperQ-650S, 650M and 650C, QAE-550C and 650S, DEAE-650M and 650C from Tosoh, QA52, DE23, DE32, DE51, DE52, DE53, Express-Ion D and Express-Ion Q from Whatman.

"Affinity chromatography" is another method of protein purification which refers to a separation technique in which a protein is reversibly and specifically bound to a biologically specific ligand, usually as a combination of spatial complementarity and one or more types of chemical interactions, e.g., electrostatic forces, hydrogen bonding, hydrophobic forces, and van der Waals forces at the binding site. These interactions are not due to the general properties of the molecule such as isoelectric point, hydrophobicity or size but are a result of specific interactions between the protein and the ligand, e.g., immunoglobulin binding to an epitope, protein A binding to immunoglobulin, interactions between a biological response modifier and its cell surface receptor. In many instances, the biologically specific ligand is also a protein or a polypeptide and can be immobilized onto a solid phase, such as the bead.

A "mixed mode ion exchange resin" is another method of protein purification and refers to a solid phase which is covalently modified with cationic, anionic or hydrophobic moieties. Examples of mixed mode ion exchange resins include BAKERBOND ABX™ (J. T. Baker; Phillipsburg, NJ), ceramic hydroxyapatite type I and II and fluoride hydroxyapatite (BioRad; Hercules, CA) and MEP and MBI HyperCel (Pall Corporation; East Hills, NY). Hydrophobic charge induction chromatography (or "HCIC") is a type of mixed mode chromatographic process in which the protein in the mixture binds to an ionizable ligand through mild hydrophobic interactions in the absence of added salts (e.g. a lyotropic salts). The mixed mode refers to one mode for binding and another mode for elution, For example, a solid phase useful in HClC contains a ligand which has the combined properties of thiophilic effect (i.e., utilizing the properties of thiophilic chromatography), hydrophobicity and an ionizable group for its separation capability. Accordingly, an adsorbent used in a method of the invention contains a ligand that is ionizable and mildly hydrophobic at neutral (physiological) or slightly acidic pH, e.g., about pH 5 to 10, preferably about pH 6 to 9.5. At this pH range, the ligand is predominantly uncharged and binds a protein via mild non-specific hydrophobic interaction. As pH is reduced, the ligand acquires charge and hydrophobic binding is disrupted by electrostatic charge repulsion towards the solute due to the pH shift. Examples of suitable ligands for use in HCIC include any ionizable aromatic or heterocyclic structure (e.g. those having a pyridine structure, such as 2- aminomethylpyridine, 3-aminomethylpyridine and 4-aminomethylpyridine, 2- mercaptopyridine, 4-mercaptopyridine or 4-mercaptoethylpyridine, mercaptoacids, mercaptoalcohols, imidazolyl based, mercaptomethylimidazole, 2- mercaptobenzimidazole, aminomethylbenzimidazole, histamine, mercaptobenzimidazole, diethylammopropylamine, aminopropyhnorpholine, aminopropylimidazole, aminocaproic acid, nitrohydroxybenzoic acid, nitrotyrosine/ethanolamine, dichlorosalicylic acid, dibromotyramine, chlorohydroxyphenylacetic acid, hydroxyphenylacetic acid, tyramine, thiophenol, glutathione, bisulphate, and dyes, including derivatives thereto.

In one embodiment, reversed phase chromatography is used, which refers to a chromatographic method that uses a non-polar stationary phase. In another embodiment, reverse phase fast protein liquid chromatography is used.

In a further embodiment, the protein purification method is performed using a basic buffer.

In a further embodiment, the protein purification method comprises, consists or consists essentially of a one step method or a two or more step method.

Methods for purifying MIS that are described in the art may also be used. By way of example, US 4,404,188 describes a method of purifying MIS which comprises chromatographing an aqueous solution of MIS on an anionic exchange resin and collecting unbound fractions comprising said biologically active MIS; and chromatographing said unbound biologically active fractions from said anionic exchange resin on a cationic resin and collecting unbound fractions comprising said biologically active MIS. US 5,310,880 describes a method for purifying MIS which comprises the steps of: (a) binding the recombinant MIS to an antibody-chromatography matrix, said antibody being specific to MIS; (b) substantially removing contaminating enzymes having MIS proteolytic activity or inhibitors of MIS antiproliferative activity by adding to the matrix an effective amount of an alkali metal halide solution wherein said solution contains an effective amount of chelating agent, and (c) recovering the recombinant MIS by eluting with an acid solution having a pH of between about 2.5 and 4.0.

In one embodiment, the method for producing MIS in a plant comprises the steps of: (a) incubating a plant into which has been introduced a nucleic acid construct comprising, consisting or consisting essentially of a first nucleic acid sequence encoding a protein that induces the formation of a protein body in a plant, optionally, further comprising a nucleic acid sequence encoding a non-naturally occurring repeat sequence motif; a second nucleic acid sequence encoding MIS; and optionally a third nucleic acid sequence encoding an amino acid linker in which a peptide bond therein can be specifically cleaved; wherein said first, second and third nucleic acid sequences are operably linked to each other; (b) incubating (for example, growing) said plant under conditions that allow for the expression of MIS as a fusion protein in said plant; (c) recovering the protein body comprising the fusion protein from the plant; (d) solubilising the pelleted fraction comprising the fusion protein; (e) releasing MIS from said fusion protein; and (f) purifying the released MIS protein.

A further aspect relates to a nucleic acid construct comprising said nucleic acid sequence and a regulatory nucleotide sequence that regulates the transcription of said nucleic acid sequence, as described herein. The construct may be a double-stranded, recombinant DNA fragment comprising one or more MIS nucleic acids.

A further aspect relates to a vector comprising the nucleic acid sequence, the nucleic acid molecule or the nucleic acid construct. Suitable vectors include, but are not limited to episomes capable of extra-chromosomal replication such as circular, double-stranded DNA plasmids; linearized double-stranded DNA plasmids; and other vectors of any origin. The vector includes a vector suitable for transforming bacteria and/or introducing into plants. The vector comprising the nucleic acid sequence, the nucleic acid molecule or the construct described herein may be a plasmid, a cosmid or a plant vector that, when introduced into a cell, is integrated into the genome of said cell and is replicated along with the chromosome (or chromosomes) in which it has been integrated. A basic bacterial or plant vector suitably comprises a broad host range replication origin; a selectable marker; and, for Agrobacterium transformations, T-DNA sequences for Agrobacterium-mediated transfer to plant chromosomes. Sequences suitable for permitting integration of the heterologous sequences into the plant genome may be used as well. These might include transposon sequences, and the like, Cre/lox sequences and host genome fragments for homologous recombination, as well as Ti sequences which permit random insertion into a plant genome.

A promoter may be incorporated into the vector to create an expression vector which may be particularly useful for expressing the fusion proteins that are described herein. Suitable expression vectors include episomes capable of extra-chromosomal replication such as circular, double-stranded DNA plasmids; linearized double-stranded DNA plasmids; and other functionally equivalent expression vectors of any origin. An expression vector comprises at least a promoter operably-linked to a MIS nucleic acid or MIS nucleic acid construct and the like. The promoter may be directly linked to the MIS nucleic acid or there may be intervening nucleic acids in between - such as nucleic acids encoding one or more components of a fusion protein.

In preparing the nucleic acid sequences, nucleic acid constructs, nucleic acid vectors and the like, the various fragments thereof may be subjected to different processing conditions, such as ligation, restriction enzyme digestion, PCR, in vitro mutagenesis, linker and adapter addition, and the like. Thus, nucleotide transitions, transversions, insertions, deletions, or the like, may be performed on the DNA which is employed in the construct for expression of MIS. Methods for restriction digests, Klenow blunt end treatments, ligations, and the like are well known to those in the art and are described, for example, by Maniatis et al. (in Molecular Cloning: A Laboratory Manual (1989) Cold Spring Harbor Laboratory Press, Cold Spring Harbor, N.Y.).

In another aspect, there is described a fusion protein comprising, consisting or consisting essentially of: (i) an amino acid sequence encoding a protein that induces the formation of a protein body in a plant, optionally, wherein said amino acid sequence further comprises an amino acid sequence encoding a non-naturally occurring repeat sequence motif; (ii) optionally an amino acid sequence encoding a linker in which a peptide bond therein can be specifically cleaved; and (iii) an amino acid sequence encoding MIS.

The fusion protein is the expression product of the nucleic acid sequence or the nucleic acid molecule described herein in a plant cell. The fusion protein is accumulated in stable, endoplasmic reticulum-derived protein bodies in a plant cell.

In a further aspect, there is described a plant or plant material comprising the nucleic acid sequence, the nucleic acid construct, the vector or the fusion protein described herein.

In a further aspect, there is described MIS obtained or obtainable by the method of the present invention.

Formulations of recombinant MIS obtained or obtainable by the present invention or protein bodies comprising MIS and having the desired degree of purity may be prepared for storage by mixing with optional pharmaceutically acceptable carriers, excipients or stabilizers (see Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as olyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes; or non-ionic surfactants such as polyethylene glycol (PEG).

Recombinant MIS protein can also be pegylated or bound to polyethylene glycol using known methods. The pegylated MIS protein may be more stable *in vivo* and have a resulting longer half-life in the body when administered to a mammal in need of treatment. Generally, the pharmaceutical compositions may be formulated and administered using methods similar to those used for other pharmaceutically important polypeptides. The recombinant MIS protein may be stored in lyophilized form, reconstituted with sterile water just prior to administration and administered intravenously. Preferably, the pharmaceutical formulation will be administered in dosages that are determined by routine dose titration experiments for the particular condition to be treated.

The following examples are provided as an illustration and not as a limitation. Unless otherwise indicated, the present invention employs conventional techniques and methods of molecular biology, plant biology and plant breeding.

### EXAMPLES

### Example 1 - Materials & Methods

### Cloning and infiltration

Nucleic acid constructs comprising nucleotide sequences encoding gamma-zein wild type gene, fragments and variants thereof are each ligated to a synthetic sequence encoding mature MIS. Where a fragment or variant of gamma-zein is used, the nucleic acid construct further comprise a nucleotide sequence encoding the native gamma-zein signal peptide at the 5' end if it is present in the fragment or variant. For certain experiments, a synthetic nucleic acid sequence encoding a linker comprising a protease cleavage site is also included in the construct, positioned between the gamma-zein and MIS coding sequences. The coding sequence of mature MIS has been optimized for expression in plants. The nucleic acid constructs are cloned into a vector at a site where a min35S promoter drives expression of the nucleic acid construct in tobacco plant cells.

Vectors comprising the cloned nucleic acid constructs are introduced into *Agrobacterium tumafaciens* strain Agl1. Agrobacterium cells are grown at 28°C and 250 rpm on a rotary shaker up to an OD600 greater than 1.6. After growth, the bacteria is collected by centrifugation at 8'000 g and 4°C for 15 min and resuspended in infiltration solution containing 10 mM MgCl2 and 5 mM (2-(n-morpholino)-ethanesulfonic acid, MES), final pH 5.6, and OD600= 2.

Plants (*Nicotiana benthamiana*) are grown under normal conditions and individual leaves are infiltrated by standard techniques using a syringe. The leaf is carefully inverted, exposing the abaxial side, and a 1-mL needleless syringe containing the bacterial suspension is used to pressure-infiltrate the leaf intracellular spaces. Six to ten days after infiltration, leaf disks are collected in a heat-sealable pouch, sealed and placed between layers of dry-ice for at least 10 minutes.

### Extraction of recombinant proteins

Tobacco leaves are ground in liquid nitrogen and homogenized using extraction buffer (50 mM Tris-HCl pH 8,200 mM dithiothreitol (DTT) and optional protease inhibitors (aprotinin, pepstatin, leupeptinc, phenylmethylsulphonyl fluoride and E64 [(N-(N-(L-3-trans-carboxyoxirane-2-carbonyl)-Lleucyl)-agmantine] per gram of fresh leaf material. The homogenates are stirred for 20 min at 4°C and then centrifuged (24000 rpm 20 min, 4°C). The material is filtered through Miracloth by gravity and then centrifuged (200 x g 10 min, 4°C), followed by further centrifugation (6000 x g 10 min, 4°C). The pelleted fraction is washed in 1% Triton X-100 and agitated for 20 minutes.

### Western blot analysis

Proteins are separated on 15% SDS polyacrylamide gel and transferred to nitrocellulose membranes (0.22 ptM) using a semidry apparatus. Membranes are incubated with gamma-zein specific antibody (Ludevid et al. (1985) Plant Sci. 41: 41-48.) and incubated with horseradish peroxidase conjugated antibodies. Immunoreactive bands are detected by enhanced chemiluminescence (ECL western blotting system, Amersham).

### ELISA assays

ELISA assays are conducted for MIS quantification on soluble leaf protein extracts and partially purified fusion proteins. Microtiter plates (MaxiSorp, Nalgene Nunc International) are loaded with soluble proteins (100 micro.I) diluted in phosphate-buffered saline pH 7.5 (PBS) and incubated overnight at 4°C. After washing the wells three times, specific binding sites are blocked with 3% bovine serum albumin (BSA) in PBS-T (PBS comprising 0. 1% Tween 20), one hour at room temperature. The plates are incubated with MIS antiserum for two hours and after four washes with PBS-T, are incubated with peroxidase-conjugated secondary antibodies for two hours. Primary and secondary antibodies are diluted in PBS-T comprising 1% BSA. After washing extensively with PBS-T, the enzymatic reaction is carried out at 37°C with substrate buffer comprising hydrogen peroxide. The reaction is stopped after 10 min with 2N sulphuric acid and the optical density is measured at 450 nm using a Multiskan EX spectrophotometer (Labsystems). The antigen concentration in plant extracts is extrapolated from a standard curve obtained by using MIS antiserum.

### Solubilisation of fusion protein

The fusion protein is incubated in the buffer chosen for solubilisation overnight at room temperature.

### Cleavage of fusion protein

For cleavage, the fusion proteins are incubated with the cleavage agent in 30 microlitres of digestion buffer (*eg*. 50 mM Tris-HCl pH8, 0.5M EDTA, 1mM DTT , 0.2% TEV) for 3 hours at 30 °C. Digestion products are analysed on 18% Tris-Tricine polyacrylamide gel electrophoresis and released MIS is detected by immunoblot.

### Purification of released MIS

MIS protein is resuspended in 20 mM Tris-HCl pH 8.6 and desalted on a PD 10 column (Sephadex G-25 M, Amersham Pharmacia). Desalted protein extracts are fractionated by reverse phase fast protein liquid chromatography using an AKTA Explorer with a RESOURCE RPC 3ml column (ID0010). A first buffer comprising 2% acetonitrile, 0.1% TCA and 20 mM beta-mercaptoethanol and a second buffer comprising 80% acetonitrile, 0.1% TCA and 20 mM beta-mercaptoethanol is used. The flow rate is adjusted to 2ml/min and a gradient of 0-60% of buffer (80% acetonitrile, 0.1% TCA and 20 mM beta-mercaptoethanol) in 10CV and 60-100% of buffer (80% acetonitrile, 0.1% TCA and 20 mM beta-mercaptoethanol) in 20m CV was used. Fractions are eluted in 1ml plus 0.5 ml volumes. The presence of MIS in eluted fractions is assessed by 15% SDS polyacrylamide gel electrophoresis and immunoblot detection using MIS antiserum. Positive fractions are desalted and concentrated with 5 K NMWL centrifugal filters (BIOMAX, Millipore).

### Example 2 - Expression levels of MIS-gamma-zein fusion protein

A gamma-zein-Enterokinase-MIS fusion protein construct (gamma-zein-MIS) is introduced into tobacco plants using Agrobacterium agroinfiltration. Total protein is extracted and quantified by Western blot using gamma-zein-specific antibody. A control experiment using MIS expressed under the same conditions without gamma-zein is also carried out (MIS). Expression levels from the average of four agroinfiltration events are as follows.

For gamma-zein-MIS, the expression levels are between about 0.2 and 0.6 g gamma-zein-MIS/kg fresh weight.

For MIS without gamma zein, the expression levels are between about 0.1 and 0.3 g gamma-zein-MIS/kg fresh weight.

Based on these average results, it is concluded that the expression of gamma-zein-MIS is about 30 times higher than the expression level without gamma-zein.

### Example 3 - Analysis of different non-naturally occurring repeat motifs in gamma-zein.

Gamma-zein-MIS fusion constructs are prepared using different non-naturally occurring repeat motifs in gamma-zein. The following constructs are tested: gamma-zein peptide only (gamma-zein-wt); gamma-zein with an (PPPVAL)n repeat motif; gamma-zein with a (PPPVEL)n repeat motif; gamma-zein with a (PPPAPA)n repeat motif and gamma-zein with a (PPPEPE)n repeat motif.

The constructs are separately infiltrated into different Tobacco plants using Agrobacterium agroinfiltration. Total protein is extracted and quantified by Western blot using gamma-zein-specific antibody. Expression levels from the average of two agroinfiltration events relative to gamma-zein-wt are as follows:

| Construct tested | Expression level |
|---|---|
| Gamma-zein-wt | 1.0 |
| Gamma-zein-(PPPVAL)n | 0.75 |
| Gamma-zein-(PPPVEL)n | 7.81 |
| Gamma-zein-(PPPAPA)n | 6.97 |
| Gamma-zein-(PPPEPE)n | 2.75 |

Three out of the five constructs tested (gamma-zein-Glu, gamma-zein-(PPPAPA)n, and gamma-zein-(PPPEPE)n significantly increase expression levels as compared to gamma-zein-wt alone.

### Example 4 - Analysis of different gamma zein repeat motifs using C-terminal MIS.

Gamma-zein-MIS fusion constructs are prepared using different non-naturally occurring repeat motifs in gamma-zein with MIS at the C-terminus of the construct The following constructs are used: gamma-zein peptide only (gamma-zein-wt); gamma-zein with an (PPPVAL)n repeat motif; gamma-zein with a (PPPVEL)n repeat motif; gamma-zein with a (PAPA)n repeat motif and gamma-zein with a (PEPE)n repeat motif.

The constructs are separately infiltrated into different Tobacco plants using Agrobacterium agroinfiltration. Total protein is extracted and quantified by Western blot using gamma-zein-specific antibody. The relative expression levels from the average of four agroinfiltration events relative to gamma-zein-wt is as follows:

| Construct tested | Expression level |
|---|---|
| Gamma-zein-wt | 1.0 |
| Gamma-zein-(PPPVAL)n | 1.16 |
| Gamma-zein-(PPPVEL)n | 6.84 |
| Gamma-zein-(PPPAPA)n | 6.46 |
| Gamma-zein-(PPPEPE)n | 3.18 |

Soluble extracts of MIS fusion proteins are obtained from leaves of transgenic tobacco plants and are then centrifuged at low speed (200 x g). The precipitated proteins are resuspended in buffer and solubilised. The yields after solubilisation relative to gamma-zein-wt are as follows:

| Construct tested | Expression level |
|---|---|
| Gamma-zein-wt | 1.0 |
| Gamma-zein-(PPPVAL)n | 3.5 |
| Gamma-zein-(PPPVEL)n | 13.5 |
| Gamma-zein-(PPPAPA)n | 4.9 |
| Gamma-zein-(PPPEPE)n | 8.7 |

Gamma-zein-(PPPVAL)n, gamma-zein-(PPPVEL)n, gamma-zein-(PPPAPA)n and gamma-zein-(PPPEPE)n significantly increases expression levels of MIS. All of the constructs allow the accumulation of the fusion protein in dense structures as seen by the recovery of the protein in the pellet after low speed centrifugation with no apparent loss of protein in the supernatant. In this experiment, gamma-zein-(PPPVEL)n-MIS results in the best yield.

### Example 5 - Recovery of protein bodies comprising fusion protein

The following method is used to recover the gamma-zein-(PPPVEL)n-MIS and gamma-zein-(PPPAPA)n-MIS fusion protein bodies.

Tobacco leaves are ground in liquid nitrogen and homogenized using extraction buffer (50 mM Tris-HCl pH 8,200 mM dithiothreitol (DTT) and optional protease inhibitors (aprotinin, pepstatin, leupeptinc, phenylmethylsulphonyl fluoride and E64 [(N-(N-(L-3-trans-carboxyoxirane-2-carbonyl)-Lleucyl)-agmantine] per gram of fresh leaf material. The homogenates are stirred for 20 min, centrifuged for 20 minutes (24000 rpm at 4°C) and agitated for 20 minutes. The mixture is filtered by gravity through one layer of Miracloth. A further centrifugation step (200 x g for 10 minutes at 4°C) to remove solids and cell debris is followed by a second centrifugation step (6000 x g for 10 minutes at 4 °C) to recover fusion protein in the pellet. This is followed by a 2 x wash with 1% Triton X-1 00 and agitation for 20 minutes followed by a further centrifugation step (1500 x g for 10 minutes at 4°C). This method also allows for the concentration and enrichment of the protein bodies.

### Example 6 - Solubilisation of fusion protein

Different sets of buffers are tested to solubilise the fusion protein. Variables tested include pH, salt, detergent, urea, reducing agent, ratio, time and temperature. A set of conditions identified in this experiment to solubilise gamma-zein-Glu-MIS and gamma-zein-PAPA-MIS protein bodies is a buffer comprising 50mM Tris pH9, about 5mM TCEP, about 50mM DTT, and about 4M urea at a ratio of protein bodies:buffer of 1:10 (w/v). The protein bodies are incubated with the buffer overnight at room temperature.

### Example 7 - Cleavage of fusion protein

A variety of different cleavage agents are analysed in order to cleave MIS and without leaving residual amino acids at the N-terminus of MIS. Cleavage is carried out for 3 hours at 30°C in 50mM Tris pH 8.0, 0.5mM EDTA, 1mM DTT and 0.2% cleavage agent. The cleavage agents tested include enterokinase, plasmin, TEV protease and intein.

In this experiment, it is found that TEV protease cleaves at the correct position in the constructs tested using a (Gly)5 linker without leaving residual amino acids on the MIS protein. The construct also comprises a PAPA or a Glu non-naturally occurring sequence motif(s).

*A Mycobacterium xenopi* Gyrase A intein is also used for the successful separation of MIS from the fusion protein.

### Example 8 -Purification of cleaved MIS protein

Various chromatographic methods are tested to identify a method that results in the desired level of purity for MIS. Reverse phase fast protein liquid chromatography is used to purify MIS under the following conditions:

| **System/Technique** | **AKTA Explorer/Reverse Phase** |
|---|---|
| Buffer A | 2% acetonitrile, 0.1% TCA and 20 mM β-mercaptoethanol |
| Buffer B | 80% acetonitrile, 0.1 % TCA and 20 mM β-mercaptoethanol |
| Column | RESOURCE RPC-3 ml-ID0010 |
| Injection | A2 pump |
| Flow Rate | 2 mL/min |
| Wash | 5 CV (5ml) |
| Gradient | 0-60% B in 10 CV + 60-100% B in 20 CV |
| Elution fraction | 1ml + 0.5 mL (microplate) |

| | |
|---|---|
| CV = column volumes. The eluted fractions are analysed by SDS-PAGE as shown in Figure 1. Fractions of purified MIS are obtained. | |

### Example 9 - MIS assay

MIS is assayed for bioactivity using, for example, the standard organ culture bioassay for MIS (Endocrinology (1992) 131, 291-296) to screen all plant generated samples for bioactivity and the results may be compared to purified MIS secreted from CHO cells (Cell (1986) 45, 685-698). Briefly, female fetal rat urogenital ridges are placed on agar-coated stainless steel grids above fortified CMRL 1066 media (GIBCO/BRL, Gaithersburg, Md.) comprising 10% female fetal calf serum and the MIS comprising sample to be assayed. After 3 days of incubation, specimens are fixed in 15% formalin, embedded in paraffin, cut in 8 mm serial sections, stained, and scored from grade 0 (no regression) to grade 5 (complete regression).

Any publication cited or described herein provides relevant information disclosed prior to the filing date of the present application. Statements herein are not to be construed as an admission that the inventors are not entitled to antedate such disclosures. All publications mentioned in the above specification are herein incorporated by reference. Various modifications and variations of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in cellular, molecular and plant biology or related fields are intended to be within the scope of the following claims.

SEQUENCES

SEQ ID NO. 1

Nucleotide sequence of human mature MIS optimised for expression in plants

tcagctggtgctaccgctgctgatgggccttgcgccttgagggagttatctgtggacctgcgagcagaaaggtcagt
tctaatacctgagacttatcaagcaaacaattgtcaaggtgtctgcggttggccccaaagtgataggaatccacgtt
atggtaaccatgtagttcttctcttgaaaatgcaagctcgtggtgctgccctagctcgaccaccctgttgtgttcct
acagcctacgccggaaagttacttatcagtttatcagaagaaagaatcagtgctcaccacgttcctaatatggtcgc
tacagagtgtggatgcagataa

SEQ ID NO. 2

Protein sequence of human mature MIS optimised for expression in plants

SAGATAADGPCALRELSVDLRAERSVLIPETYQANNCQGVCGWPQSDRNPRYGNHVVLLLKMQARGAALARPPCCVP
TAYAGKLLISLSEERISAHHVPNMVATECGCR

SEQ ID NO. 3

DNA sequence of Zea mays gamma zein (Genbank Accession No. NM_001111884)

1 gcaccagttt caacgatcgt cccgcgtcaa tattattaaa aaactcttac atttctttat
61 aatcaacccg cactcttata atctcttctc tactactata ataagagagt ttatgtacaa
121 aataaggtga aattatgtat aagtgttctg gatattggtt gttggctcca tattcacaca
181 acctaatcaa tagaaaacat atgttttatt aaaacaaaat ttatcatata tcatatatat
241 atatatacat atatatatat atatataaac cgtagcaatg cacgggcata taactagtgc
301 aacttaatac atgtgtgtat taagatgaat aagagggtat ccaaataaaa aacttgttcg
361 cttacgtctg gatcgaaagg ggttggaaac gattaaatct cttcctagtc aaaattgaat
421 agaaggagat ttaatctctc ccaatcccct tcgatcatcc aggtgcaacc gtataagtcc
481 taaagtggtg aggaacacga aacaaccatg cattggcatg taaagctcca agaatttgtt
541 gtatccttaa caactcacag aacatcaacc aaaattgcac gtcaagggta ttgggtaaga
601 aacaatcaaa caaatcctct ctgtgtgcaa agaaacacgg tgagtcatgc cgagatcata
661 ctcatctgat atacatgctt acagctcaca agacattaca aacaactcat attgcattac
721 aaagatcgtt tcatgaaaaa taaaataggc cggacaggac aaaaatcctt gacgtgtaaa
781 gtaaatttac aacaaaaaaa aagccatatg tcaagctaaa tctaattcgt tttacgtaga
841 tcaacaacct gtagaaggca acaaaactga gccacgcaga agtacagaat gattccagat
901 gaaccatcga cgtgctacgt aaagagagtg acgagtcata tacatttggc aagaaaccat
961 gaagctgcct acagccgtct cggtggcata agaacacaag aaattgtgtt aattaatcaa

1021 agctataaat aacgctcgca tgcctgtgca cttctccatc accaccactg ggtcttcaga
1081 ccattagctt tatctactcc agagcgcaga agaacccgat cgacaccatg agggtgttgc
1141 tcgttgccct cgctctcctg gctctcgctg cgagcgccac ctccacgcat acaagcggcg
1201 gctgcggctg ccagccaccg ccgccggttc atctaccgcc gccggtgcat ctgccacctc
1261 cggttcacct gccacctccg gtgcatctcc caccgccggt ccacctgccg ccgccggtcc
1321 acctgccacc gccggtccat gtgccgccgc cggttcatct gccgccgcca ccatgccact
1381 accctactca accgccccgg cctcagcctc atccccagcc acacccatgc ccgtgccaac
1441 agccgcatcc aagcccgtgc cagctgcagg gaacctgcgg cgttggcagc accccgatcc
1501 tgggccagtg cgtcgagttc ctgaggcatc agtgcagccc gacggcgacg ccctactgct
1561 cgcctcagtg ccagtcgttg cggcagcagt gttgccagca gctcaggcag gtggagccgc
1621 agcaccggta ccaggcgatc ttcggcttgg tcctccagtc catcctgcag cagcagccgc
1681 aaagcggcca ggtcgcgggg ctgttggcgg cgcagatagc gcagcaactg acggcgatgt
1741 gcggcctgca gcagccgact ccatgcccct acgctgctgc cggcggtgtc ccccactgaa
1801 gaaactatgt gctgtagtat agccgctggc tagctagcta gttgagtcat ttagcggcga
1861 tgattgagta ataatgtgtc acgcatcac

SEQ ID NO. 4

Translated amino acid sequence of SEQ ID No. 3.

MRVLLVALALLALAASATSTHTSGGCGCQPPPPVHLPPPVHLPPPVHLPPPVHLPPPVHLPPPVHLPPPVHVPPPVH
LPPPPCHYPTQPPRPQPHPQPHPCPCQQPHPSPCQLQGTCGVGSTPILGQCVEFLRHQCSPTATPYCSPQCQSLRQQ
CCQQLRQVEPQHRYQAIFGLVLQSILQQQPQSGQVAGLLAAQIAQQLTAMCGLQQPTPCPYAAAGGVPH

SEQ ID NO. 5

Amino acid sequence of a fragment of gamma-zein

MRVLLVALALLALAASATSTHTSGGCGCQPPPPVHLPPPVHLPPPVHLPPPVHLPPPVHLPPPVHLPPPVHVPPPVH
LPPPPCHYPTQPPRPQPHPQPHPCPCQQPHPSPCQ

SEQ ID No. 6

Amino acid sequence of (PPPAPA)n

APAPPPAPAPPPAPAPPPAPAPPPAPAPPPAPAPPPAPAPPPA

SEQ ID No. 7

Amino acid sequence of (PPPEPE)n

EPAPPPEPEPPPEPEPPPEPEPPPEPEPPPEPEPPPEPEPPPE

SEQ ID No. 8

Amino acid sequence of (PPPVEL)n

VELPPPVELPPPVELPPPVELPPPVELPPPVELPPPVEVPPPVE

SEQ ID No. 9

Amino acid sequence of (PPPVAL)n

VALPPPVALPPPVALPPPVALPPPVALPPPVALPPPVAVPPPVA

SEQ ID No. 10

Amino acid sequence of (PPPVTL)n

VTLPPPVTLPPPVTLPPPVTLPPPVTLPPPVTLPPPVTVPPPVT

SEQ ID No. 11

Amino acid sequence of (PPPAPA)n

PPPAPAPPPAPAPPPAPCPCPAPAPPPCP

SEQ ID No. 12

Amino acid sequence of (PPPEPE)n

PPPEPEPPPEPEPPPEPCPCPEPEPPPCP

## Claims

1. A method for producing Mullerian Inhibitor Substance in a plant comprising incubating or growing a plant into which has been introduced or infiltrated a nucleic acid construct comprising, consisting or consisting essentially of a nucleic acid sequence encoding a Mullerian Inhibitor Substance fusion protein that comprises a fusion protein partner that induces the formation of a protein body in a plant, preferably, wherein the step of introducing or infiltrating the plant is performed prior to the incubating or growing step.

2. The method according to claim 1, wherein the nucleic acid construct comprises:
a first nucleic acid sequence encoding a fusion protein partner that induces the formation of a protein body in a plant, optionally, further comprising a nucleic acid sequence encoding one or more non-naturally occurring repeat sequence motifs;
a second nucleic acid sequence encoding Mullerian Inhibitor Substance; and
optionally a third nucleic acid sequence encoding an amino acid linker in which a peptide bond therein can be specifically cleaved;
wherein said first, second and third nucleic acid sequences are operably linked to each other.

3. The method according to claim 1 or claim 2, wherein said fusion protein partner that induces the formation of a protein body in a plant is gamma-zein or a fragment thereof.

4. The method according to any of the preceding claims, wherein the nucleic acid sequence encoding the fusion protein partner that induces the formation of a protein body in a plant further comprises a nucleic acid sequence encoding a peptide that directs the Mullerian Inhibitor Substance fusion protein towards the endoplas mic reticulum of a plant cell, preferably a signal peptide.

5. The method according to any of the preceding claims, comprising the additional step of:
recovering the protein body comprising the Mullerian Inhibitor Substance fusion protein from the plant, preferably wherein said step comprises the steps of:
(i) homogenising the plant material;
(ii) centrifuging the homogenised plant material at low speed, preferably, about 200 x g;
(iii) centrifuging the homogenised plant material at a higher speed than step (ii), preferably, about 6000 x g; and
(iv) recovering the protein bodies comprising the Mullerian Inhibitor Substance fusion protein in the pelleted fraction.

6. The method according to any of the preceding claims, comprising the further step of:
solubilising the fusion protein, preferably, wherein said solubilisation step comprises the use of a mixture comprising, consisting or consisting essentially of urea, dithiothreitol and (tris(2-carboxyethyl)phosphine) .

7. The method according to any of the preceding claims, comprising the further step of:
releasing Mullerian Inhibitor Substance from said Mullerian Inhibitor Substance fusion protein, preferably, wherein a protease, preferably, TEV protease, or a protein splicing means, preferably an intein, is used to release Mullerian Inhibitor Substance from said fusion protein

8. The method according to claim 7, wherein said protease or said protein splicing means cleaves Mullerian Inhibitor Substance from the Mullerian Inhibitor Substance fusion protein without leaving any residual amino acids at the cleaved end of Mullerian Inhibitor Substance.

9. The method according to any of the preceding claims, comprising the further step of:
purifying the released Mullerian Inhibitor Substance protein.

10. A nucleic acid construct comprising, consisting or consisting essentially of:
a first nucleic acid sequence encoding a fusion protein partner that induces the formation of a protein body in a plant, optionally, wherein said sequence comprises a nucleic acid sequence encoding a non-naturally occurring repeat sequence motif;
a second nucleic acid sequence encoding Mullerian Inhibitor Substance; and
optionally a third nucleic acid sequence encoding an amino acid linker in which a peptide bond therein can be specifically cleaved;
optionally a second nucleic acid sequence encoding a linker in which a peptide bond therein can be specifically cleaved; and
a third nucleic acid sequence encoding Mullerian Inhibitor Substance wherein said first, second and third nucleic acid sequences are operably linked to each other.

11. The nucleic acid construct according to claim 10, further comprising a regulatory nucleotide sequence that regulates the transcription of said nucleic acid sequences.

12. A vector comprising the nucleic acid construct according to claim 10 or claim 11.

13. A Mullerian Inhibitor Substance fusion protein comprising, consisting or consisting essentially of:
(i) an amino acid sequence encoding a fusion protein partner that induces the formation of a protein body in a plant, optionally, wherein said amino acid sequence further comprises an amino acid sequence encoding one or more non-naturally occurring repeat sequence motifs;
(ii) optionally an amino acid sequence encoding a linker in which a peptide bond therein can be specifically cleaved;
(iii) an amino acid sequence encoding MIS.

14. A transgenic plant or plant material derived therefrom comprising the nucleic acid construct according to claim 10 or claim 11 or the vector according to claim 12, or the Mullerian Inhibitor Substance fusion protein according to claim 13.

15. A plant protein body comprising the Mullerian Inhibitor Substance fusion protein according to claim 14.
